# EUROPÄISCHE PATENTSCHRIFT

(11) **EP 1 764 122 B1**
(45) Veröffentlichungstag und Bekanntmachung des Hinweises auf die Patenterteilung: **14.09.2011**
(21) Anmeldenummer: 05020157.3
(22) Anmeldetag: 15.09.2005
(51) Int. Cl.: A61M 25/00, A61M 5/158, A61M 5/32

(54) **Insertionskopf mit Nadelschutz im Griff**
Injection head with needle protection in the handle
Tête d'injection avec une protection d'aiguille dans la poignée

(43) Veröffentlichungstag der Anmeldung: 21.03.2007
(73) Patentinhaber: F.Hoffmann-La Roche AG, 4070 Basel (CH); Roche Diagnostics GmbH, 68305 Mannheim (DE)
(72) Erfinder: Scheurer, Simon, 3006 Bern (CH)
(74) Vertreter: Wess, Wolfgang

(56) Entgegenhaltungen:
- EP-A- 0 615 768
- WO-A-02/081012
- WO-A-2005/046781
- DE-U1-202004 017 862
- FR-A- 2 752 164

## Beschreibung

Die Erfindung betrifft einen Insertionskopf für medizinische oder pharmazeutische Anwendungen, der auf einem organischen Gewebe, vorzugsweise der menschlichen Haut, platzierbar ist und eine Einführeinrichtung aufweist, die in das Gewebe eindringt, wenn der Insertionskopf auf dem Gewebe platziert wird oder gegebenenfalls auch erst nachdem der Insertionskopf auf dem Gewebe platziert wurde. Der Insertionskopf kann insbesondere Bestandteil eines Infusionssets für die Verabreichung eines Medikaments sein.

Aus der DE 198 21 723 ist ein Insertionskopf bekannt mit einer Basis, die eine auf organischem Gewebe platzierbare Unterseite aufweist, über die eine mit der Basis unbeweglich verbundene, flexible Kanüle vorragt, die bei Platzierung der Basis auf der Oberfläche des Gewebes in das Gewebe eindringt. Die Kanüle wird für ein Einstechen in das Gewebe von einer Einstechnadel stabilisiert, welche die Kanüle durchragt. Um einen Benutzer vor Verletzungen durch die vorstehende Einstechnadel und die Einstechnadel mit der Einführeinrichtung vor Beschädigungen zu schützen, ist an der Unterseite des Insertionskopfs ein zylindrischer Nadelschutz lösbar befestigt. Der Nadelschutz benötigt Raum, und es besteht die Gefahr, dass er sich bei unachtsamer Handhabung von dem Insertionskopf löst. Zum Entfernen des Nadelschutzes benötigt der Benutzer ferner beide Hände, wobei sich die eine Hand nach Entfernen des Nadelschutzes noch in unmittelbarer Nähe der dann frei vorstehenden Einstechnadel befindet.

Aus der deutschen Patentanmeldung Nr. 10 2004 039 408.3 ist ein Insertionskopf mit einer Einführeinrichtung bekannt, die aus einer Schutzposition, in der sie in einer Aufnahme aufgenommen ist, in eine Einführposition bewegbar ist, in der sie über die Unterseite des Insertionskopfs vorragt und bei dem Aufsetzen des Insertionskopfs auf der Gewebeoberfläche in das Gewebe eindringt. Die Aufnahme ist integraler Bestandteil des Insertionskopfs, der hierfür einen zur Unterseite offenen Hohlraum bildet. In der Schutzposition ist die Einführeinrichtung vollständig in dem die Aufnahme bildenden Hohlraum aufgenommen, insbesondere ragt ihr freies Ende nicht über die Unterseite oder seitlich vor. Der Insertionskopf benötigt vorteilhafterweise zwar keinen gesonderten Schutz für die Einführeinrichtung und ist deshalb im Auslieferungszustand so flach wie im Gebrauchszustand, d.h. wie in dem auf dem Gewebe platzierten Zustand. Wegen der integriert gebildeten Aufnahme verlängert sich jedoch der Insertionskopf.

Die DE 20 2004 017 862 U1 bezieht sich auf eine Auszieheinrichtung zum geschützten Herausziehen einer flexiblen Punktionsnadel aus einem Katheter, insbesondere einem Katheter mit flexiblem Verweilschlauch, nach dem Punktieren, mit einem Verbindungsabschnitt zum Verbinden der Auszieheinrichtung mit dem Katheter in einem Gebrauchsanfangszustand, einer Gleitschiene und einem in der Gleitschiene linear bewegbaren Gleiter, wobei der Gleiter einen Mitnehmerabschnitt aufweist und wobei die Punktionsnadel mit einem aus dem Katheter hervorragenden Ende an der Gleitschiene derart festgelegt/festlegbar ist, dass sie mit einer Schlaufe um den Mitnehmerabschnitt des Gleiters herumgeführt ist und an diesem anliegt.

Die WO 02/081012 A2 bezieht sich auf eine Vorrichtung zum Einbringen einer Kanüle in Gewebe mit einer Kanüle mit einem Schutzelement, welches die Kanüle aufnehmen kann, einem Betätigungselement, mit welchem die Kanüle aus dem Schutzelement herausbewegt werden kann und einer Halterung, welche mit der Kanüle fest verbunden ist sowie einer Vorrichtung zum Einbringen einer Kanüle in Gewebe mit einer Kanüle, einer Kanülenbewegungsvorrichtung zum Herausbewegen der Kanüle und einem Rückzugelement, welches mit der Kanülenausschubvorrichtung gekoppelt ist, um die Kanülenausschubvorrichtung nach Ausschieben der Kanüle wieder zurückzuziehen sowie einem System zum Verbinden einer Flüssigkeitszufuhr mit einer Kanüle.

Die FR 2 752 164 bezieht sich auf eine Nadel, um Haut zu durchstechen, z.B. um eine Kammer unterhalb der Haut zu implantieren. Die Nadel ist mit einem Zuführrohr verbunden und drehbar in einem Gehäuse gelagert, und zwar zwischen einer Position, um die Haut zu durchstechen und einer eingezogenen Position. Ein Druckknopf wir in ein Ende des Gehäuses bewegt, und zwar durch den Zeigefinger, und bewegt die Nadel. Die Nadel ist an einer Welle befestigt, die sich in dem Gehäuse dreht. Die Welle hat zwei Nocken, und eine Nocke wird durch den Knopf bewegt, um die Welle zu drehen und die Nadel aus dem Gehäuse zu bewegen. Die andere Nocke wird bewegt, wenn der Knopf freigegeben ist und die Nadel einzieht.

Es ist eine Aufgabe der vorliegenden Erfindung, einen Insertionskopf zu schaffen, der gefahrlos handhabbar ist, und im Gebrauchszustand, d.h. nach Platzierung auf einer Gewebeoberfläche und eingedrungener Einführeinrichtung, kompakt ist.

Diese Aufgabe wird erfindungsgemäß durch den Gegenstand des Patentanspruchs 1, und insbesondere durch dessen kennzeichnende Merkmale erfüllt. Die Unteransprüche beschreiben bevorzugte Ausführungsformen des erfindungsgemäßen Insertionskopfes.

Ein Insertionskopf, wie die Erfindung ihn betrifft, umfasst eine Basis mit einer auf organischem Gewebe platzierbaren Unterseite, eine in das Gewebe einführbare Einführeinrichtung und eine Aufnahme, die zumindest ein freies Ende der Einführeinrichtung in einer Schutzposition der Einfiihreinrichtung aufnimmt.

Nach der Erfindung ragt in der Schutzposition der Einführeinrichtung zumindest deren freies Ende nicht über die Unterseite der Basis vor. Vielmehr wird die Einführeinrichtung von der Basis beweglich gelagert, so dass sie relativ zu der Basis aus der Schutzposition in eine Einführposition bewegbar ist, in der ihr freies Ende über die Unterseite vorsteht. Vorzugsweise steht die Einführeinrichtung in der Schutzposition über ihre gesamte Länge hinter der Unterseite der Basis zurück und wird vollständig abgeschirmt, vorzugsweise auch blickdicht verdeckt. In bevorzugten Ausführungen weist die Einführeinrichtung in der Schutzposition zumindest im Wesentlichen parallel zu der Unterseite bzw. der Auflagefläche der Basis. Dies begünstigt eine flache Bauweise der Basis, wobei deren Höhe rechtwinklig zur Unterseite gemessen wird. Ferner ist die Aufnahme mit der Basis lösbar verbunden. Im verbundenen Zustand schafft die Aufnahme den Schutz für die Einführeinrichtung, so dass die Basis von dieser Funktion entlastet ist und daher bei der Basis die sonst zur Erfüllung der Schutzfunktion erforderliche Länge eingespart wird. Die Aufnahme ist zu der Unterseite der Basis hin offen. Grundsätzlich würde eine Öffnung nur für die Einführeinrichtung genügen, vorzugsweise umgibt die lösbare Aufnahme jedoch die Basis oder einen Teil der Basis topf- oder schüsselartig, vorzugsweise der Form nach wie ein halber Topf oder eine halbe Schüssel. Die Aufnahme kann nach Lösen einer Verbindung vorzugsweise einfach nach oben von der Basis abgenommen werden.

Zur Erleichterung der Handhabung ist der Insertionskopf vorteilhafterweise mit einem von der Basis abragenden Griff ausgestattet. Der Griff oder im Falle eines bevorzugt mehrteiligen Griffes zumindest eine Griffkomponente kann mit der Basis lösbar verbunden sein. Vorzugsweise bildet ein solcher Griff oder eine solche Griffkomponente die lösbare Aufnahme für die Einführeinrichtung. In weiter bevorzugter Ausführung weist der Griff einen von der Oberseite der Basis aufragenden Griffteil auf, von dem die Aufnahme seitlich abragt.

Vorzugsweise ist der Griff mehrteilig und umfasst eine Griffkomponente, die relativ zu der Basis und einer weiteren Griffkomponente oder gegebenenfalls mehreren weiteren Griffkomponenten beweglich ist. Die bewegliche Griffkomponente ist mit der Einführeinrichtung vorzugsweise so gekoppelt, dass eine Bewegung dieser Griffkomponente die Bewegung der Einführeinrichtung bewirkt. Eine weitere Griffkomponente, die vorzugsweise ein Widerlager für das Bewegen der beweglichen Griffkomponente bildet, kann relativ zu der Basis im verbundenen Zustand unbeweglich sein und vorteilhafterweise die lösbare Aufnahme bilden. Grundsätzlich kann stattdessen die bewegliche Griffkomponente die lösbare Aufnahme bilden. Mehrere Griffkomponenten können die Aufnahme auch erst gemeinsam bilden.

Aufgrund der Ausstattung des Griffs mit einer beweglichen Griffkomponente kann die Bewegung der Einführeinrichtung allein durch Greifen und Betätigen des Griffs bewirkt werden. Der Griff bildet selbst das Widerlager für die bewegbare Griffkomponente. In diesem Sinne wird hier der das Widerlager bildende Teil des Griffs als weitere Griffkomponente bezeichnet. Die bewegliche Griffkomponente kann beispielsweise als Druckknopf gebildet sein. Die weitere Griffkomponente kann ein Gehäuse sein, aus dem solch ein Druckknopf herausragt. In ebenfalls bevorzugten Ausführungen bilden die beiden Griffkomponenten erst gemeinsam den Griff, beispielsweise die Hälften eines insgesamt zweiteiligen Griffs.

Für die automatisierte Platzierung mittels des erfindungsgemäßen Inserters weist der Insertionskopf, vorzugsweise dessen Griff, eine Haltestruktur auf, die mit der Halteeinrichtung des Inserters in dem Halteeingriff ist. Falls der Griff wie bevorzugt eine mit der Basis unbeweglich verbundene Griffkomponente und eine relativ zu dieser Griffkomponente bewegliche Griffkomponente aufweist, bildet vorzugsweise die unbewegliche Griffkomponente die Haltestruktur. Die Haltestruktur kann an dem Griff oder der unbeweglichen Griffkomponente insbesondere in einem Stück angeformt sein, vorzugsweise ist sie mit dem Griff oder der unbeweglichen Griffkomponente zumindest steif verbunden.

Die bewegliche Griffkomponente bildet vorzugsweise das vorstehend in Bezug auf das System aus Insertionskopf und Inserter genannte Empfangsglied, wird jedoch nachfolgend als bewegliche Griffkomponente bezeichnet, solange besonders bevorzugte Ausführungen des Insertionskopfs beschrieben werden. Grundsätzlich muss das Empfangsglied des Systems jedoch keine Griffkomponente sein, sondern kann ausschließlich der Kopplung zwischen dem Aktivierungsglied und der Einführeinrichtung dienen.

In bevorzugten Ausführungen ist die bewegliche Griffkomponente zumindest im Wesentlichen parallel zu der Unterseite der Basis bewegbar. Insbesondere kann es sich um eine Linearbeweglichkeit handeln. Alternativ zu einer reinen Translationsbeweglichkeit kann die zweite Komponente beispielsweise schwenkbeweglich angebracht sein.

Die wenigstens eine weitere Griffkomponente ist mit der Basis vorzugsweise nicht beweglich verbunden. Grundsätzlich wäre es jedoch auch denkbar, dass beide Griffkomponenten relativ zu der Basis beweglich mit dieser verbunden sind.

Für die Übertragung der Bewegung der beweglichen Griffkomponente auf die Einführeinrichtung kann eine steife Kopplung vorgesehen sein, d.h. die bewegliche Griffkomponente und die Einführeinrichtung können steif miteinander verbunden sein, worunter auch eine Urformung in einem Stück verstanden wird. Eine steife Kopplung kann beispielsweise im Falle einer bevorzugten Schwenkbeweglichkeit der Einführeinrichtung ohne weiteres dann verwirklicht werden, wenn auch die bewegliche Griffkomponente schwenkbeweglich ist. Gegenüber dem Insertionskopf der deutschen Patentanmeldung Nr. 10 2004 039 408.3 weist der erfindungsgemäße Insertionskopf dennoch den Vorteil auf, dass die weitere Griffkomponente dem Benutzer als Widerlager dienen kann und nicht das Gewebe über die Basis die für die Schwenkbewegung aufzubringende Kraft aufnehmen muss, vielmehr wird diese Kraft durch das Halten der weiteren Griffkomponente vom Benutzer aufgenommen. Verfügt der Benutzer über einen Inserter, insbesondere den erfindungsgemäßen, mit dem der Insertionskopf auf dem Gewebe platziert und dabei die Einführrichtung eingeführt wird, nimmt der Inserter diese Kraft auf.

In bevorzugten Ausführungen sind die bewegliche Griffkomponente und die Einführeinrichtung über ein Getriebe miteinander gekoppelt. Eine derartige Kopplung hat den Vorteil, dass die Beweglichkeit der Griffkomponente nicht der Beweglichkeit der Einführeinrichtung entsprechen muss, sondern beide Beweglichkeiten jeweils einzeln für sich optimal gestaltet werden können. So kann die Einführeinrichtung insbesondere schwenkbeweglich und die bewegliche Griffkomponente translatorisch beweglich und dabei vorzugsweise linear geführt sein. Im Falle einer Schwenkbeweglichkeit auch der Griffkomponente kann deren Schwenkachse eine andere als die der Einführeinrichtung sein. Während die Einführeinrichtung vorzugsweise um eine zu der Unterseite der Basis zumindest im wesentlichen parallelen Rotationsachse schwenkbar ist, was im übrigen für sämtliche Ausführungsformen der beweglichen Griffkomponente gilt, kann eine schwenkbewegliche Griffkomponente um eine zur Unterseite zumindest im wesentlichen rechtwinkligen Rotationsachse schwenkbeweglich sein. Ein Getriebe kann aber auch dann von Vorteil sein, wenn im Falle einer schwenkbeweglichen Griffkomponente deren Rotationsachse von der Rotationsachse der schwenkbeweglichen Einführeinrichtung parallel beabstandet ist. In solch einem Fall kann der Schwenkwinkel der Griffkomponente mittels eines Getriebes untersetzt oder vorzugsweise übersetzt auf die Einführeinrichtung übertragen werden.

Eine bevorzugte getriebetechnische Kopplung umfasst ein Zahnrad und eine Zahnstange, die miteinander in einem Zahneingriff sind und bei Bewegung der Griffkomponente miteinander kämmen. Die Zahnstange ist vorzugsweise mit der beweglichen Griffkomponente verbunden, so dass eine Bewegung der Griffkomponente in Längsrichtung der Zahnstange in eine Drehbewegung des in diesem Fall mit der Einführeinrichtung verbundenen Zahnrads übertragen wird. Bei entsprechend feiner Verzahnung bzw. Zahnteilung kann ein vergleichsweise kurzer Hub der beweglichen Griffkomponente in eine Drehbewegung des Zahnrads über einen beträchtlichen Teil einer vollen Umdrehung, vorzugsweise in eine viertel Umdrehung des Zahnrads, übertragen werden. Vorteilhafterweise ist die bewegliche Griffkomponente mit der Zahnstange in einem Stück geformt. Anstatt einer Kopplung über ein oder gegebenenfalls mehrere Zahnpaarungen, kann die Kopplung auch als Führungskurve und Eingriffsglied gebildet sein oder ein solches Führungskurvengelenk umfassen, obgleich eine Kopplung mittels Zahneingriffs der Vorzug gegeben wird.

Der Griff ist in bevorzugten Ausführungen wie bereits erwähnt lösbar mit der Basis verbunden. Bevorzugt löst sich diese Verbindung bei der Bewegung der Einführeinrichtung in die Einführposition automatisch, besonders bevorzugt bei der Bewegung der beweglichen Griffkomponente. Alternativ wäre es grundsätzlich jedoch ebenfalls denkbar, den Griff oder die Basis mit einer weiteren beweglichen Komponente auszustatten, durch deren Betätigung die Verbindung mit der Basis gelöst wird. Die Verbindung zwischen dem Griff und der Basis kann zwar durch einen reinen Reibschluss hergestellt sein, bevorzugter beruht sie jedoch ausschließlich auf einem Formschluss oder einer Kombination aus Form- und Reibschluss. Um die Verbindung zu schaffen, sind die Basis und der Griff je mit wenigstens einem Verbindungselement ausgestattet, die bei bestehender Verbindung miteinander in einem Eingriff sind. Um die Verbindung lösen zu können, ist vorzugsweise wenigstens eines der Verbindungselemente gegen eine rückstellende Elastizitätskraft aus dem Eingriff bewegbar. In bevorzugten Ausführungen dient die bewegliche Griffkomponente nicht nur der Überführung der Einführeinrichtung in die Einführposition, sondern auch dem Lösen der Verbindung, indem die zweite Griffkomponente bei ihrer Bewegung durch Kontakt, vorzugsweise Gleitkontakt, eines der Verbindungselemente gegen die Elastizitätskraft aus dem Eingriff bewegt, beispielsweise elastisch abbiegt. Obgleich weniger bevorzugt, kann die weitere Griffkomponente jedoch alternativ in einem Stück mit der Basis geformt oder unlösbar an der Basis befestigt sein. In solch einer Ausführung sollte sie jedoch möglichst kurz sein.

Falls der Insertionskopf nicht über einen besonderen, d.h. ausgeprägten Griff verfügt oder die lösbare Aufnahme nicht Bestandteil eines derartigen Griffs ist, beispielsweise indem sie an dem Griff in einem Stück angeformt ist, wird es dennoch bevorzugt, wenn durch die für das Lösen der Aufnahme erforderlichen Handgriffe automatisch auch die Einführeinrichtung aus der Schutzposition in die Einführposition bewegt wird. So kann an der lösbaren Aufnahme beispielsweise ein Druckknopf oder ein anderes Betätigungselement angebracht oder ein solches Element mit der Aufnahme geeignet verbunden sein und die Aufnahme durch Betätigung des Betätigungselements von der Basis gelöst werden.

Die Einführeinrichtung kann eine biegesteife Kanüle oder Nadel sein. Bevorzugt ist die Einführeinrichtung zumindest in dem Gewebe flexibel. Insbesondere kann die Einführeinrichtung eine Biegesteifigkeit aufweisen, die sich im eingeführten Zustand aufgrund einer zwischen dem Material der Einführeinrichtung und dem umgebenden Gewebe stattfindenden Wechselwirkung verringert. Die Einführeinrichtung kann alternativ auch in herkömmlicher Weise flexibel gebildet sein, beispielsweise als flexible Kanüle, und von einer biegesteifen Einstecheinrichtung während der Einführung in das Gewebe stabilisiert werden. Die Einführeinrichtung ist vorzugsweise in eine Einführrichtung langgestreckt und vorzugsweise schlank.

Falls die Einführeinrichtung von Hause aus flexibel ist, d.h. nicht erst durch Wechselwirkung mit dem Gewebe flexibel wird, wird sie vorzugsweise mittels einer Einstecheinrichtung stabilisiert, um zu verhindern, dass die Einführeinrichtung bei dem Einführen in das Gewebe knickt. Die Einstecheinrichtung kann insbesondere als dünne Einstechnadel gebildet sein. Wenn die Einführeinrichtung in das Gewebe eingeführt worden ist, wird die Einstecheinrichtung vorteilhafterweise entfernt. Das Entfernen einer derartigen Einstecheinrichtung wird vorzugsweise durch Entfernen der lösbaren Aufnahme bewirkt, beispielsweise mit dem Griff bewerkstelligt. Falls die Einstecheinrichtung wie bevorzugt in der Schutzposition noch nicht mit der lösbaren Aufnahme verbunden ist, verbindet sie sich bei der Bewegung in die Einführposition vorzugsweise automatisch mit der Aufnahme oder einem damit verbundenen Teil. Hierfür kann an ihrem von dem freien Ende der Einführeinrichtung abgewandten Ende ein Verbindungselement, vorgesehen sein, das zugleich mit dem Abschluss der Bewegung in die Einführposition oder kurz zuvor mit einem Verbindungsgegenelement, das mit der lösbaren Aufnahme verbunden ist in einen Verbindungseingriff gelangt. Die Verbindung kann zwar grundsätzlich rein reibschlüssig sein, vorzugsweise umfasst sie jedoch einen Formschluss zumindest. Das Verbindungselement der Einstecheinrichtung kann mit dem Verbindungsgegenelement insbesondere eine Schnappverbindung bilden. Grundsätzlich genügt für eine formschlüssige Verbindung auch nur ein einfacher Hintergriff bezüglich der Richtung, in welche die lösbare Aufnahme von der Basis entfernt werden soll; ein elastischer Schnappeingriff ist daher nicht unumgänglich erforderlich.

Die Einführeinrichtung ragt in der Einführungsposition vorzugsweise von der Unterseite des Gehäuses vor. Grundsätzlich kann sie jedoch stattdessen auch von einer Seite des Gehäuses vorragen, solange sie für ein Eindringen in das Gewebe über die Unterseite ausreichend weit vorragt. Die Einführeinrichtung ragt in der Einführposition vorzugsweise mit einer an subkutane Applikationen angepassten Länge über die Unterseite des Gehäuses vor, vorzugsweise unmittelbar von der Unterseite ab oder aus der Unterseite hervor. Für Applikationen innerhalb der Haut oder in intramuskulärem Gewebe ist die Einführeinrichtung entsprechend kürzer oder länger. Als Einführeinrichtung wird derjenige Längenabschnitt verstanden, der in der Applikation in das Gewebe ragt.

In bevorzugten Ausführungen ist die Einführeinrichtung mit der Basis bewegbar permanent verbunden. Dass die Einführeinrichtung mit der Basis permanent verbunden ist, bedeutet, dass sie zumindest für die Bewegung aus der Schutzposition in die Einführposition und/oder aus der Einführposition in die Schutzposition nicht von der Basis gelöst wird. Vorzugsweise ist die Einführeinrichtung sogar in dem Sinne permanent mit der Basis verbunden, dass sie von der Basis gar nicht gelöst werden kann, zumindest nicht ohne eine besondere Kraftanstrengung oder nur durch Zerstörung.

Vorzugsweise ist die Einführeinrichtung mit der Basis so verbunden, dass die Einführeinrichtung relativ zu der Basis schwenkbar ist. Vorteilhafterweise, jedoch nur optional, kann die Einführeinrichtung oder zumindest deren freies Ende nach einem Ausschwenken wieder in die lösbare Aufnahme eingeschwenkt werden. In derartigen Ausbildungen bilden die Basis und ein mit der Einführeinrichtung verbundenes Gelenkelement ein Drehgelenk. Die Einführeinrichtung ragt von dem Gelenkelement vorzugsweise einfach quer zu der Rotationsachse des Gelenks ab, beispielsweise indem sie an das Gelenkelement angeformt ist, oder sie kann grundsätzlich auch erst wieder gelenkig mit dem Gelenk verbunden sein. In der Lage, welche die Einführeinrichtung gerade einnimmt, wenn ihr freies Ende über die Unterseite vorschwenkt, schließt die Längsachse der Einführeinrichtung mit der Unterseite einen spitzen Winkel von vorzugsweise weniger als 50° ein. Vorzugsweise ist der Winkel kleiner als 30°, so dass die Längsachse bzw. die Einführeinrichtung im Moment des Ausschwenkens zumindest im Wesentlichen parallel zu der Unterseite bzw. der Auflagefläche der Basis weist. Die Längsachse der Einführeinrichtung, die um die Rotationsachse geschwenkt wird, schneidet die Rotationsachse vorzugsweise. Falls die Längsachse der Einführeinrichtung die Rotationsachse nicht schneidet, sondern in einem Abstand kreuzt, ist der Abstand vorzugsweise deutlich kleiner als die Länge der Einführeinrichtung. Vorzugsweise ist der Abstand höchstens halb so groß wie die Eindringtiefe bzw. Länge der Einführeinrichtung. Der Schwenkwinkel der Einführeinrichtung beträgt in bevorzugten Ausführungen 90° ± 10°. In ebenfalls vorteilhaften Ausführungen kann der Schwenkwinkel jedoch auch kleiner sein, insbesondere falls die Einführeinrichtung in der Einführposition nicht rechtwinklig zur Unterseite der Basis weist, sondern in einem spitzen Winkel, der allerdings wenigstens 30° betragen sollte. Entsprechend beträgt der Schwenkwinkel in derartigen Ausführungen vorzugsweise wenigstens etwa 30° oder jeden Zwischenwert zwischen etwa 30° und etwa 90°. Grundsätzlich kann der Schwenkwinkel auch größer als 90° sein.

In einer alternativen Ausführungsform kann die Basis für die Einführeinrichtung eine Verschiebeführung bilden, an der die Einführeinrichtung bei ihrer Bewegung aus der Schutzposition in die Einführposition und/oder aus der Einführposition in die Schutzposition translatorisch geführt wird. In einer derartigen Ausführung bilden die Basis und ein Gelenkelement, das mit der Einführeinrichtung verbunden ist, ein Schubgelenk.

Auch von einem derartigen Gelenkelement ragt die Einführeinrichtung in bevorzugter Ausführung einfach ab, vorzugsweise in Schubrichtung. In noch einer Alternative weist die Einführeinrichtung oder eine sie stabilisierende Einstecheinrichtung eine derartige Biegesteifigkeit auf, die es ermöglicht, die Einführeinrichtung oder gegebenenfalls eine sie stabilisierende Einstecheinrichtung mit dem jeweils freien Ende zu der Unterseite der Basis hin in die Aufnahme zu biegen, so dass das freie Ende in die Aufnahme hineinragt und in der Aufnahme mit der Basis lösbar verrastet oder anderweitig in diesem Biegezustand gegen ihre rückstellende Elastizität lösbar gehalten wird. Eine derartige Ausführung ist besonders dann denkbar, wenn die Einführeinrichtung in der Einführposition mit der Unterseite der Basis einen spitzen Winkel einschließt. Denkbar ist auch, dass für ein derartiges Einbiegen die Einführeinrichtung ein Stück weit hinter der Unterseite der Basis an der Basis oder der lösbaren Aufnahme befestigt ist, um bereits innerhalb eines Hohlraums des Insertionskopfs eine gewisse Biegelinie zu erhalten. Andererseits ist es vorteilhaft, wenn die Einführeinrichtung in der Einführposition unmittelbar oder möglichst nahe von der die Gewebeoberfläche im Gebrauch kontaktierenden Unterseite vorragt, d.h. an der Unterseite oder in einem möglichst kurzen Abstand von der Unterseite so eingespannt ist, vorzugsweise fest oder gegebenenfalls axial beweglich und ansonsten fest, das eine beim Eindringen in das Gewebe biegbare Länge der Einführeinrichtung oder einer sie stabilisierenden Einstecheinrichtung möglichst kurz ist. Eine in der Einführposition unmittelbar von oder nahe der Unterseite abragende Einführeinrichtung wird jedenfalls dann bevorzugt, wenn die Einführeinrichtung für die Schutzposition nicht verbogen wird.

Obgleich es genügt, wenn die Einführeinrichtung nur in eine Richtung, d.h. einmalig, relativ zu der Basis bewegbar ist, dann vorzugsweise aus der Schutzposition in die Einführposition, kann es vorteilhaft sein, wenn die Einführeinrichtung zwischen den beiden Positionen hin und her bewegbar ist, so dass die Einführeinrichtung für einen Gebrauch in die Einführposition und für eine Entsorgung wieder in die Schutzposition bewegt werden kann. Die Verbindung zwischen der Einführeinrichtung und dem Gehäuse ist oder umfasst vorzugsweise einen Formschluss, der insbesondere ein Gelenk bilden kann. Die Verbindung kann alternativ auch rein kraftschlüssig oder sogar rein stoffschlüssig sein, beispielsweise dann, wenn die Einführeinrichtung in der Schutzposition gebogen ist.

Der Insertionskopf ist vorzugsweise Bestandteil eines Infusionssets für die Verabreichung von Insulin, eines Schmerzmittels oder eines anderen per Infusion verabreichbaren Medikaments oder ist für eine derartige Verwendung vorgesehen. Anstatt für eine Medikamentenverabreichung oder grundsätzlich auch eines anderen verabreichbaren Produkts kann der Insertionskopf auch zu Diagnosezwecken dienen. In solchen Applikationen kann die Einführeinrichtung Träger eines Sensors zum Messen von beispielsweise der Glukosekonzentration in einer Körperflüssigkeit oder einer anderen physikalischen und/oder biochemischen Größe dienen, die für den Gesundheitszustand eines Patienten maßgeblich ist oder sein kann. Der Insertionskopf kann zu Diagnosezwecken auch als Perfusionsvorrichtung gebildet sein. In solch einer Ausbildung wird die Einführeinrichtung nach dem Einführen in das Gewebe von einer Spülflüssigkeit durchströmt, die ein oder mehrere bestimmte Inhaltsstoffe der Körperflüssigkeit bei dem Durchströmen aufnimmt, um die mit dem betreffenden Inhaltsstoff oder die mehreren Inhaltsstoffen angereicherte Spülflüssigkeit zu analysieren. Schließlich kann der Insertionskopf eine Vorrichtung für die Verabreichung eines Produkts und eine Diagnoseeinrichtung in Kombination bilden. Die Einführeinrichtung kann für die Zuführung eines Produkts, dass insbesondere ein Medikament oder eine Spülflüssigkeit sein kann, oder die Abführung einer Körperflüssigkeit oder nur eines oder mehrerer Inhaltsstoffe einer Körperflüssigkeit angepasst geformt sein. In derartigen Applikationen bildet die Einführeinrichtung wenigstens einen Strömungsquerschnitt. Die Einführeinrichtung kann der Zu- und Abführung von Stoffen auch in Kombination dienen. Ist der Insertionskopf nur als Messvorrichtung gebildet, so kann die Einführeinrichtung auch nur dazu dienen, einen Sensor oder einen Teil eines Sensors zu platzieren, d.h. rein als mechanische Einführeinrichtung. In einer Weiterbildung als Messvorrichtung kann der Insertionskopf über das mechanische Eindringen hinaus auch der Übertragung von Steuersignalen zu dem Sensor und/oder von Messsignalen von dem Sensor dienen. In kombinierten Applikationen kann er schließlich über wenigstens einen Strömungsquerschnitt für den Stofftransport, d.h. eine Strömungsleitung, und wenigstens eine Signalleitung verfügen. Auf die Signalleitung kann verzichtet werden, wenn der Sensor für den drahtlosen Empfang von Steuersignalen und/oder das drahtlose Senden von Messsignalen eingerichtet ist. Schließlich kann die Einführeinrichtung auch zwei oder mehr Einführelemente ausweisen, die separat abragen. So kann ein erstes Einführelement den Stofftransport in das Gewebe und ein anderes, zweites Einführelement dem Stofftransport aus dem Gewebe oder nur dem Eindringen eines Sensors oder eines Teils eines Sensors dienen. Mit mehreren Einführabschnitten, die je einen Strömungsabschnitt aufweisen, können mit dem gleichen Insertionskopf auch unterschiedliche Stoffe verabreicht werden. Dies kann auch mit einer Einführeinrichtung verwirklicht werden, die mehrere, separate Strömungsquerschnitte in einem gemeinsamen Abschnitt bildet.

Bevorzugte Merkmale werden auch in den Unteransprüchen und deren Kombinationen offenbart.

Nachfolgend werden Ausführungsbeispiele der Erfindung anhand von Figuren erläutert. An den Ausführungsbeispielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: einen Insertionskopf eines ersten Ausführungsbeispiels mit in Schutzposition befindlicher Einführeinrichtung,
- Figur 2: den Insertionskopf mit in Einführposition befindlicher Einführeinrichtung,
- Figur 3: einen Griff des Insertionskopfs des ersten Ausführungsbeispiels,
- Figur 4: eine Basis des Insertionskopfs des ersten Ausführungsbeispiels mit in Einführposition befindlicher Einführeinrichtung,
- Figur 5: den Griff der Figur 3 in einer Ansicht
- Figur 6: die Basis mit Einführeinrichtung der Figur 4 in einer Ansicht,
- Figur 7: einen Insertionskopf eines zweiten Ausführungsbeispiels mit in Schutzposition befindlicher Einführeinrichtung,
- Figur 8: den Insertionskopf des zweiten Ausführungsbeispiels mit in Einführposition befindlicher Einführeinrichtung,
- Figur 9: einen Griff des Insertionskopfs des zweiten Ausführungsbeispiels,
- Figur 10: eine Basis des Insertionskopfs des zweiten Ausführungsbeispiels mit in Einführposition befindlicher Einführeinrichtung,

- Figur 11: ein System aus einem Insertionskopf und einem Inserter eines ersten Ausführungsbeispiels vor einer Aktivierung,
- Figur 12: das System des ersten Ausführungsbeispiels nach der Aktivierung,
- Figur 13: ein System aus einem Insertionskopf und einem Inserter eines zweiten Ausführungsbeispiels vor einer Aktivierung des Insertionskopfs,
- Figur 14: das System des zweiten Ausführungsbeispiels nach der Aktivierung und
- Figur 15: das System des zweiten Ausführungsbeispiels nach einer Platzierung des Insertionskopfs auf einer Gewebeoberfläche.

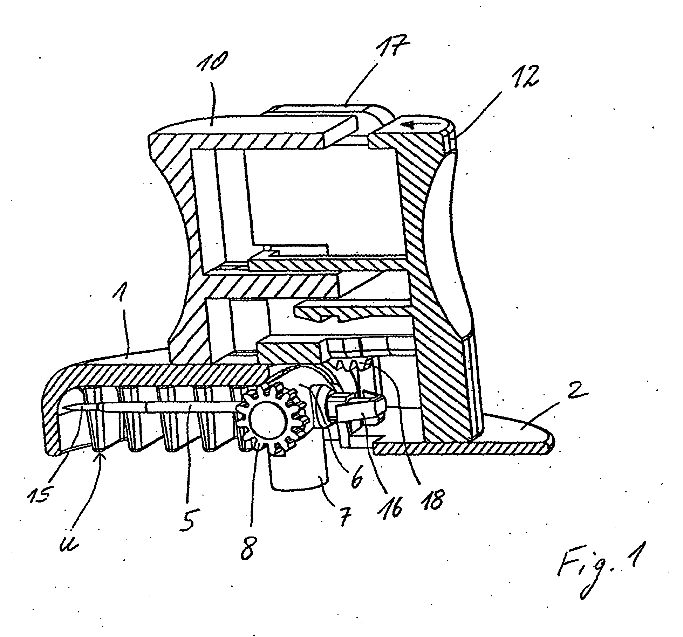

Figur 1 zeigt einen Insertionskopf eines ersten Ausführungsbeispiels in einem Längsschnitt. Der Insertionskopf umfasst eine Basis mit einem Aufnahmeteil bzw. einer Aufnahme 1 und einem Flachteil 2, die in einem Stück aus Kunststoff geformt sind. Die Basis 1, 2 ist mit ihrer Unterseite U auf organischem Gewebe platzierbar. Der Insertionskopf umfasst des Weiteren einen zweiteiligen Griff mit einer ersten Griffkomponente 10 und einer zweiten Griffkomponente 12. Die Griffkomponente 10 ist mit der Basis unbeweglich, aber lösbar verbunden. Die Griffkomponente 12 ist an der Griffkomponente 10 beweglich gehalten, wobei die Griffkomponente 12 sowohl relativ zu der Griffkomponente 10 als auch zu der Basis 1, 2 linear verschiebbar ist. Die Achse der Bewegbarkeit der Griffkomponente 12 weist parallel zu einer Unterseite U der Basis 1, 2. Die Richtung der Bewegbarkeit ist auf der Oberseite der Griffkomponente 12 mit einem Pfeil angedeutet.

Die Basis 1, 2 lagert eine Einführeinrichtung 5 schwenkbeweglich um eine zur Unterseite U parallele Rotationsachse. Die Einführeinrichtung 5 ist lang gestreckt. Im Ausführungsbeispiel ist sie als flexible Kanüle gebildet. Die Einführeinrichtung 5 wird von einer Einstecheinrichtung 15 durchragt, die als dünne Nadel gebildet ist, deren Biegesteifigkeit ausreicht, um die Einstecheinrichtung 15 gemeinsam mit der umgebenden, sich anschmiegenden Einführeinrichtung 5 durch die Hautoberfläche in subkutanes Gewebe einzustechen und dadurch die Einführeinrichtung 5 einzuführen. In bevorzugten Ausführungen ist an der Unterseite U ein Klebepad für eine Fixierung des Insertionskopfs auf dem Gewebe, vorzugsweise der Hautoberfläche, angebracht.

Für die Schwenkbeweglichkeit der Einführeinrichtung 5 und damit gemeinsam der Einstecheinrichtung 15 sorgt ein Gelenkelement 6, das eine Welle eines Drehgelenks mit der Rotationsachse als Gelenkachse bildet. Die Basis 1, 2 bildet das andere Gelenkelement des Drehgelenks in Form einer Buchse oder gegebenenfalls auch eines offenen Lagerauges. Auf der Rotationsachse des Gelenks ist zu beiden Seiten des Gelenkelements 6 je ein außenverzahntes Zahnrad 8 angeordnet und drehsteif mit dem Gelenkelement 6 verbunden, beispielsweise in einem Stück geformt. Das eine der beiden Zahnräder 8 ist in Figur 1 erkennbar. Das andere sitzt auf der gegenüberliegenden Seite des Gelenkelements 6 und wird von dem Aufnahmeteil 1 der Basis 1, 2 verdeckt. Die Einstecheinrichtung 15 durchragt das Gelenkelement 6. An das Gelenkelement 6 schließt sich eine Zuführung 7 für eine Medikamentenflüssigkeit, beispielsweise Insulin, an. Die Zuführung 7 ragt in etwa rechtwinklig zur Einführeinrichtung 5 von dem Gelenkelement 6 ab. Das Gelenkelement 6 bildet mit der Zuführung 7, den Zahnrädern 8, der Einführeinrichtung 5 und der Einstecheinrichtung 15 eine in Bezug auf die Drehbewegung des Gelenkelements 6 und der Zahnräder 8 und der Schwenkbewegung der genannten weiteren Komponenten eine Einheit.

Die bewegliche Griffkomponente 12 ist mit zwei Zahnstangen 18 versehen, die je mit einem der Zahnräder 8 in Zahneingriff sind. Von den beiden Zahnstangen 18 ist nur die mit dem verdeckten Zahnrad zusammenwirkende erkennbar. Eine ebensolche Zahnstange 18 wirkt mit dem erkennbaren Zahnrad 8 zusammen. Im Falle einer Verschiebung der Griffkomponente 12 in die durch den Richtungspfeil angedeutete Richtung, wobei die erste Griffkomponente 10 bei dieser Bewegung die Griffkomponente 12 führt, kämmen die beiden Zahnstangen 18 mit den beiden Zahnrädern 8, so dass die Verschiebebewegung der Griffkomponente 12 in eine Drehbewegung des Gelenkelements 6 und eine Schwenkbewegung der Einführeinrichtung 5 und der Einstecheinrichtung 15 sowie der Zuführung 7 übertragen wird.

Die Schwenkbewegung überführt die Einführeinrichtung 5 aus deren in Figur 1 gezeigten Schutzposition in eine Einführposition. In der Schutzposition weisen die Einführeinrichtung 5 und die Einstecheinrichtung 15 zumindest im Wesentlichen parallel zu der Unterseite U der Basis 1, 2. Die Einführeinrichtung 5 und der in die gleiche Richtung über das Gelenkelement 6 hinausstehende Abschnitt der Einstecheinrichtung 15 sind in der gemeinsamen Schutzposition in einem von dem Aufnahmeteil bzw. der Aufnahme 1 mit Ausnahme der Unterseite U umschlossenen Hohlraum aufgenommen. Mit in Schutzposition befindlicher Einführeinrichtung 5 ist gewährleistet, dass der Benutzer sich nicht an der Einstecheinrichtung 15 verletzen und umgekehrt die Einführeinrichtung 5 und die Einstecheinrichtung 15 nicht durch unachtsame Handhabung beschädigt werden können. Die Aufnahme 1 bildet bevorzugt auch einen Sichtschutz, so dass der Benutzer die Einstecheinrichtung 15 von der Oberseite des Insertionskopfs und auch bei seitlichem Blickwinkel nicht erkennen kann. Ein an der Unterseite bevorzugterweise angebrachtes Klebepad ist mit einem Durchtrittsschlitz für die Einführeinrichtung 5 und die Einstecheinrichtung 15 versehen.

Die Zahnstangen 18 sind jeweils an einer der Unterseite U zugewandten Unterseite zweier biegesteifer Zungen geformt, die von einem Seitenteil der Griffkomponente 12 in Bewegungsrichtung vorragen. Über die beiden die Zahnstangen 18 bildenden Zungen hinaus ragt von dem Seitenteil der Griffkomponente 12 wenigstens eine weitere Zunge in Bewegungsrichtung vor, die der Linearführung der beweglichen Griffkomponente 12 an einer von der Griffkomponente 10 gebildeten Führung dient.

Um die Einführeinrichtung 5 in das Körpergewebe bis unter oder gegebenenfalls nur in die Haut einzuführen, greift der Benutzer den Insertionskopf am Griff zwischen Daumen und Zeigefinger. Die Griffkomponenten 10 und 12 sind je mit einer entsprechend geformten, seitlichen Einbuchtung versehen. Durch Zusammendrücken der Griffkomponenten 10 und 12 wird die bewegliche Griffkomponente 12 bis gegen einen von der ersten Griffkomponente 10 gebildeten Anschlag gedrückt. Bei dieser Bewegung kämmen die beiden Zahnstangen 18 mit den Zahnrädern 8, so dass die Translationsbewegung der Griffkomponente 12 in eine Drehbewegung des Gelenkelements 6 und somit in eine Schwenkbewegung der Einführeinrichtung 5 und der Einstecheinrichtung 15 übertragen wird. Der Weg der Griffkomponente 12, der Durchmesser der Zahnräder 8 und die Feinheit der Verzahnungen sind so gewählt, dass eine Bewegung der Griffkomponente 12 um wenige Millimeter, beispielsweise 4 oder 5 mm, eine Schwenkbewegung der Einführeinrichtung und der Einstecheinrichtung 15 um einen Schwenkwinkel von zumindest im wesentlichen 90° in eine Einführposition bewirkt, in der die Einführeinrichtung 5 und die Einstecheinrichtung 15 über die Unterseite U der Basis 1, 2 zumindest in etwa rechtwinklig vorragen.

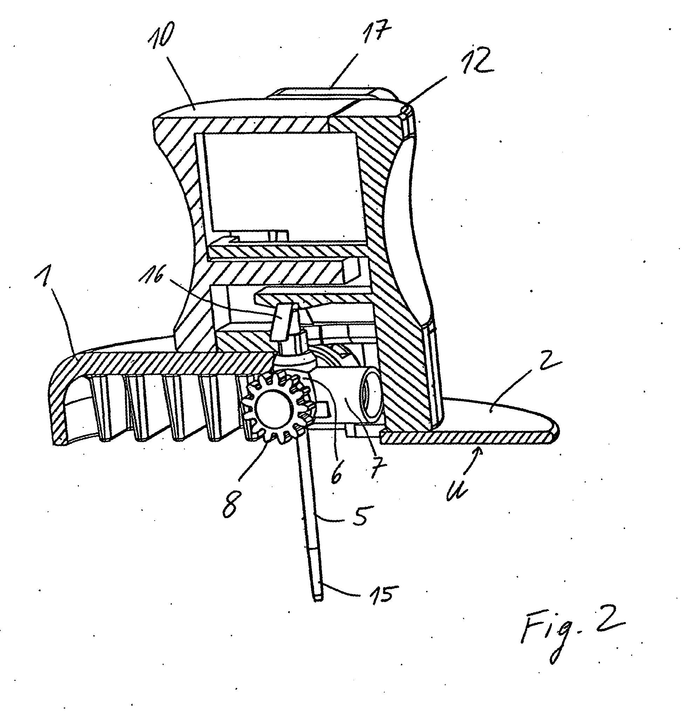

Figur 2 zeigt den Insertionskopf mit der in Einführposition befindlichen Einführ- und Einstecheinrichtung 5, 15.

Die Einstecheinrichtung 15 hat sich am Ende der Schwenkbewegung mit dem Griff 10, 12 verbunden, im Ausführungsbeispiel mit der beweglichen Griffkomponente 12. In der Schutzposition (Figur 1) besteht zwischen der Einstecheinrichtung 15 und dem Griff 10, 12 kein Kontakt, so dass die Einstecheinrichtung 15 gemeinsam mit der Einführeinrichtung 5 frei schwenken kann. Zur Herstellung der Verbindung ist an einem in der Einführposition proximalen Ende der Einstecheinrichtung 15, mit dem die Einstecheinrichtung 15 über das Gelenkelement 6 hinaussteht, ein Verbindungselement 16 angeordnet, im Ausführungsbeispiel befestigt. Das Verbindungselement 16 weist einen oder zwei abstehende Flügel auf, mit denen es ein Verbindungsgegenelement der Griffkomponente 12 in Bezug auf die Längsrichtung der Einstecheinrichtung 15 hintergreift. Das Verbindungsgegenelement der beweglichen Griffkomponente 12 ist als entsprechend hintergreifbare Schulterfläche gebildet.

Für die Platzierung des Insertionskopfs auf einer Gewebeoberfläche und der Einführung der Einführeinrichtung 5 in das Gewebe hält der Benutzer den Insertionskopf am Griff 10, 12 und bewegt ihn auf die Gewebeoberfläche zu. Dabei durchsticht die Einstecheinrichtung 15 die Gewebeoberfläche, vorzugsweise die menschliche Haut, und dringt in die Haut ein. Die sich anschmiegende Einführeinrichtung 5 dringt gemeinsam mit der Einstecheinrichtung 15 ein, bis der Insertionskopf mit seiner Unterseite U auf der Gewebeoberfläche aufsetzt und sich dabei adhäsiv auf der Hautoberfläche fixiert, vorzugsweise mittels eines Klebepads. Für die Verabreichung des Medikaments wird die Einstecheinrichtung 15 entfernt und die Zuführung 7 über einen mit der Zuführung 7 zusammenwirkenden Konnektor mit einem Medikamentenreservoir, vorzugsweise einer Medikamentenpumpe, verbunden. Um dies zu ermöglichen, wird zuvor der Griff 10, 12 von der Basis 1, 2 gelöst. Das Lösen ist nur nach dem Zusammendrücken bzw.

Zusammenschieben der Griffkomponenten 10 und 12 möglich. Allerdings löst sich die Verbindung bei der Bewegung der Griffkomponente 12 automatisch, so dass der Griff 10, 12 in die proximale Richtung, in Figur 2 nach oben, abgezogen werden kann. Bei der geraden Abziehbewegung gleitet die Einstecheinrichtung 15 durch die Einführeinrichtung 5 und das Gelenkelement 6 und gibt dadurch den Strömungsquerschnitt der Einführeinrichtung 5 frei, so dass der Strömungsquerschnitt nach Herausziehen der Einstecheinrichtung 15 auch gleichzeitig mit der Zuführung 7 fluidisch verbunden ist. Diesbezüglich kann der Insertionskopf wie beispielsweise in der DE 198 21 723 C1 und der DE 10 2004 039 408.3 beschrieben ausgebildet sein.

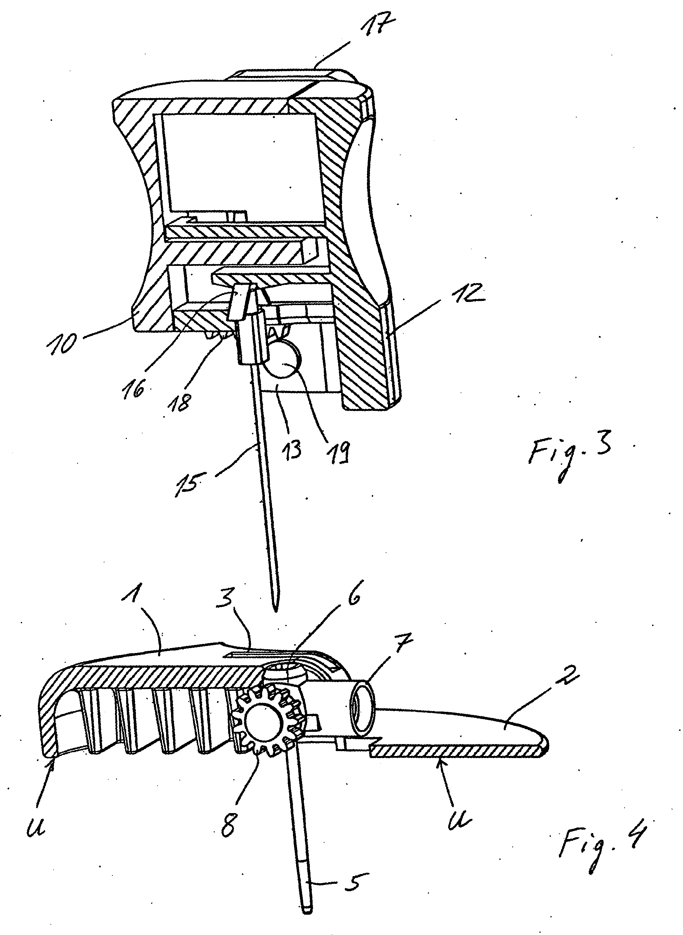

Die Figuren 3 und 4 zeigen die beiden voneinander gelösten Teile des Insertionskopfs, nämlich die Basis 1, 2 mit der Einführeinrichtung 5 einerseits und den Griff 10, 12 mit der Einstecheinrichtung 15 andererseits, in zueinander ausgerichteter Position, in der die Längsachse der Einführeinrichtung 5 und die Längsachse der Einstecheinrichtung 15 miteinander fluchten. In Figur 3 ist auch eine Ausnehmung 3 in der Basis 1, 2 erkennbar, in die im verbundenen Zustand bei Bewegung der Griffkomponente 12 eine der beiden Zahnstangen 18 einfährt und dabei mit dem in der Ausnehmung 3 angeordneten Zahnrad 8 kämmt. Die Ausnehmung 3 ist schlitzförmig. Ferner ist ein Verbindungselement 19 des Griffs 10, 12 erkennbar, das im verbundenen Zustand formschlüssig in ein Verbindungsgegenelement der Basis 1, 2 eingreift und so den Griff 10, 12 an der Basis 1, 2 hält und in Kombination mit Kontaktflächen des Griffs 10, 12 und der Basis 1, 2 relativ zu der Basis 1, 2 fixiert. Das Verbindungselement 19 ragt stummelartig von einer im distalen Bereich von der Griffkomponente 10 abragenden, elastischen Lasche 13 in eine zur Unterseite U der Basis 1, 2 parallel weisende Richtung vor und im verbundenen Zustand in eine Ausnehmung der Basis 1, 2 hinein, beispielsweise ein zum Verbindungselement 19 kongruent geformtes Loch, so dass eine Bewegung des Griffs 10, 12 in Längsrichtung des Einstechabschnitts 15 bei bestehender Verbindung verhindert wird. Um diese Verbindung zu lösen, ragt von dem Seitenteil der beweglichen Griffkomponente 12 eine nicht dargestellte weitere Zunge vor, die bei der Bewegung der Griffkomponente 12 zwischen die Basis 1, 2 und die das Verbindungselement 19 tragende Lasche 13 fährt und die Lasche 13 von der Basis 1, 2 ein Stück weit abbiegt, das jedoch ausreicht, die formschlüssige Verbindung zwischen dem Verbindungselement 19 und dem Verbindungsgegenelement zu lösen, so dass der Griff 10, 12 mit der Einstecheinrichtung 15 in deren Längsrichtung von der Basis 1, 2 abgezogen werden kann.

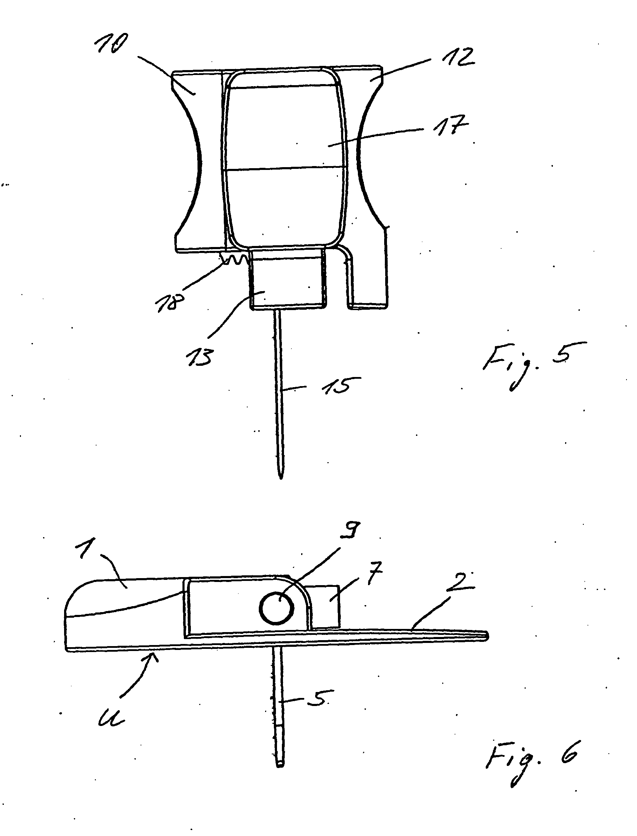

Die Figuren 5 und 6 zeigen nochmals die voneinander gelösten Teile des Insertionskopfs in einer Ansicht auf die in den Figuren 1 bis 4 abgewandte Rückseite. In dieser Ansicht ist in Figur 6 insbesondere das Verbindungsgegenelement 9 der Basis 1, 2 erkennbar, das im verbundenen Zustand, d.h. im Eingriff mit dem Verbindungselement 19, den Griff 10, 12 an der Basis 1, 2 hält.

Die in den Figuren 4 und 6 einzeln dargestellte Basis 1, 2, die Träger der Einführeinrichtung 5 und der damit eine Schwenkeinheit bildenden Teile 6, 7 und 8 ist, verbleibt auf der Gewebeoberfläche und ist in diesem Sinne ein Verbleibteil. Der Griff 10, 12 hingegen, der nun als Träger für die Einstecheinrichtung 15 dient, wird entsorgt oder gegebenenfalls wieder von der Einstecheinrichtung 15 gelöst und einer erneuten Verwendung zugeführt, während die Einstecheinrichtung 15 entsorgt wird. Das Verbleibteil 1-9 kann somit vorteilhaft flach sein und stört nicht, wenn es unter der Kleidung getragen wird. Die Flexibilität der Einführeinrichtung 5 ist derart, dass die Einführeinrichtung 5 im eingeführten Zustand als nicht störend empfunden wird, aber doch ausreichend stabil ist, um eine Versorgung mit dem Medikament sicher zu gewährleisten.

Der Griff 10, 12 kann auch in solchen Ausführungen des Insertionskopfs verwendet werden, in denen die Einführeinrichtung nicht wie die Einführeinrichtung 5 von Hause aus flexibel ist, sondern ohne Fremdstabilisierung für das Einstechen ausreichend biegesteif ist. In derartigen Ausführungen kann die zusätzliche Einstecheinrichtung 15 entfallen. Der Griff 10, 12 dient in derartigen Ausführungen ausschließlich der Handhabung des Insertionskopfs, nicht jedoch als Träger einer stabilisierenden Einstecheinrichtung 15. Eine derart modifizierte Einführeinrichtung 5 kann insbesondere als Einstechkanüle mit einem Hohlquerschnitt oder als Einstechnadel mit einem vollen Querschnitt und einem oder mehreren Strömungskanälen am äußeren Umfang gebildet sein, die nach der Einführung durch Wechselwirkung mit dem Gewebe flexibler wird.

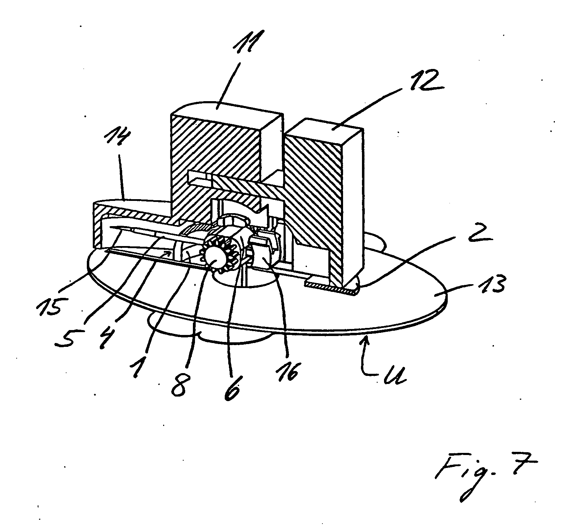

Die Figuren 7 bis 10 zeigen ein zweites Ausführungsbeispiel eines Insertionskopfs. Von den nachfolgend beschriebenen Unterschieden abgesehen entspricht der Insertionskopf des zweiten Ausführungsbeispiels dem Insertionskopf des ersten Ausführungsbeispiels.

So ist beispielhaft ein an der Unterseite U befestigtes Klebepad dargestellt, wie es auch an dem Insertionskopf des ersten Ausführungsbeispiels angebracht sein könnte.

Gegenüber dem ersten Ausführungsbeispiel sind die Aufnahme 1 und die erste Griffkomponente 11 modifiziert. Im Unterschied zum ersten Ausführungsbeispiel sind die Einführeinrichtung 5 und die Einstecheinrichtung 15 nur über einen kurzen Abschnitt in der vom Aufnahmeteil gebildeten Aufnahme 1 aufgenommen. Im zweiten Ausführungsbeispiel bildet der Griff, genauer gesagt dessen Griffkomponente 11, eine Aufnahme 14 für die Einführeinrichtung 5 und die Einstecheinrichtung 15. Die Aufnahme 1 ist seitlich mit einer zur Unterseite U offenen Ausnehmung 4 in Form eines Schlitzes versehen, durch welche die Einführeinrichtung 5 und die Einstecheinrichtung 15 in der Schutzposition aus der Aufnahme 1 hinausragen. Die Aufnahme 1 ist ihrerseits in der Aufnahme 14 aufgenommen. Die Aufnahme 14 ist zur Unterseite U hin offen, umschließt aber ansonsten die Einführeinrichtung 5 und die Einstecheinrichtung 15, vorzugsweise blickdicht.

Um die Einführeinrichtung 5 und die Einstecheinrichtung 15 aus der Schutzposition in die Einführposition zu schwenken, führt der Benutzer die anhand des ersten Ausführungsbeispiels beschriebenen Handgriffe durch, d.h. er drückt die bewegliche Griffkomponente 12 gegen die modifizierte Griffkomponente 11. Bei dem kämmenden Zahneingriff schwenken die Einführeinrichtung 5 und Einstecheinrichtung 15 in der Ausnehmung 4 aus der Aufnahme 1 und insbesondere aus der Aufnahme 14 in die Einführposition.

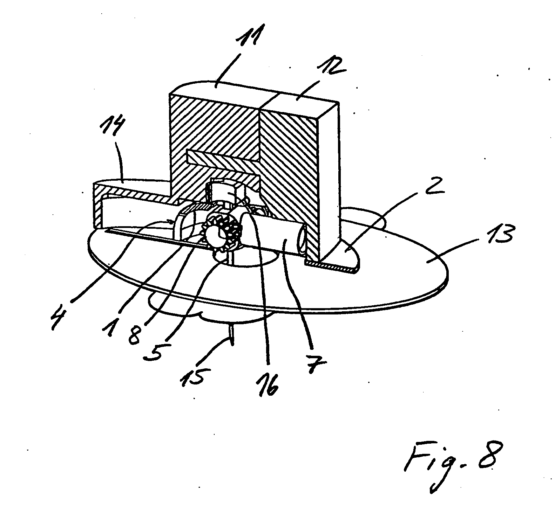

Figur 8 zeigt den Insertionskopf des zweiten Ausführungsbeispiels mit in Einführposition befindlicher Einführeinrichtung 5 und Einstecheinrichtung 15.

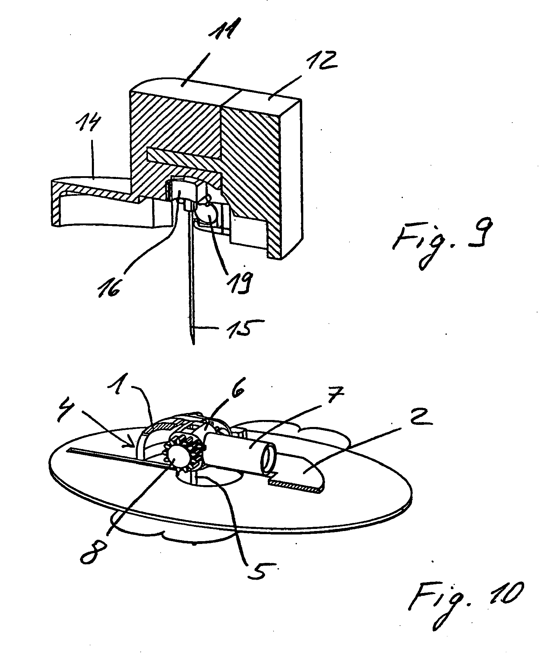

Die Figuren 9 und 10 entsprechend bezüglich des zweiten Ausführungsbeispiels den Figuren 3 und 4 zum ersten Ausführungsbeispiels. Wie jedoch nicht zuletzt der Blick auf Figur 10 zeigt, ist die Basis 1, 2 des zweiten Ausführungsbeispiels gegenüber der Basis 1, 2 des ersten Ausführungsbeispiels vorteilhaft verkürzt, da nicht mehr sie, sondern der Griff 11, 12 für die Einführeinrichtung 5 und die Einstecheinrichtung 15 in deren Schutzposition die Schutzfunktion übernimmt.

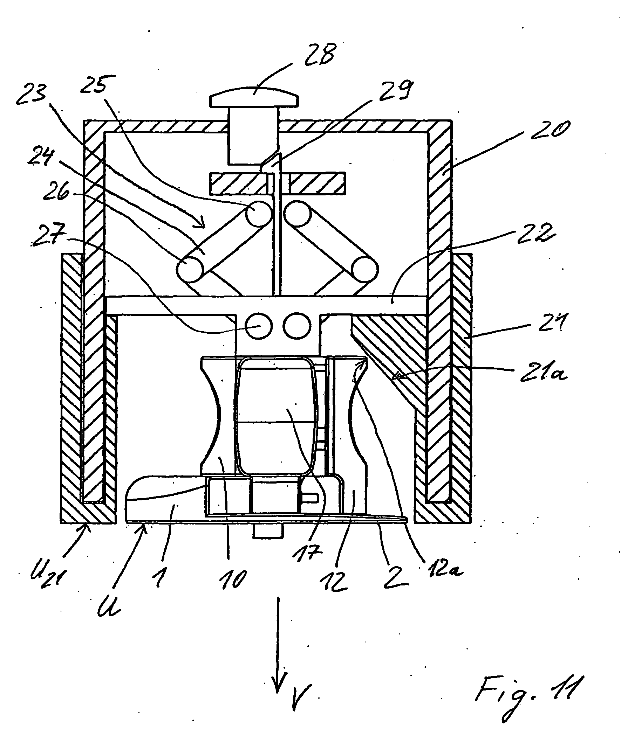

Figur 11 zeigt ein System eines ersten Ausführungsbeispiels bestehend aus dem Insertionskopf des ersten Ausführungsbeispiels und einem Inserter, welcher der Platzierung des Insertionskopfs auf dem Gewebe dient, so dass der Benutzer den Insertionskopf bei der Platzierung nicht zwischen den Fingern greifen muss. Insbesondere hält der Benutzer bei der Überführung der Einführeinrichtung 5 in die Einführposition den Insertionskopf nicht am Griff. Diese Aktivierung des Insertionskopfs wird mit Hilfe des Inserters vorgenommen. Der Benutzer wird daher noch sicherer vor Stichverletzungen und die Einführeinrichtung 5 sowie die Einstecheinrichtung 15 werden noch sicherer vor Beschädigungen durch unachtsame Handhabung geschützt, nämlich durch den Inserter.

Der Inserter weist ein Insertergehäuse 20 auf, das als Hülsenteil mit einem Boden gebildet ist und von außen betrachtet im Wesentlichen die Form eines Topfs aufweist. Das Insertergehäuse 20 nimmt eine Halteeinrichtung und einen Antrieb für den Insertionskopf auf. Die Halteeinrichtung umfasst eine Haltefeder, beispielsweise eine Blattfeder, die den Insertionskopf in der in Figur 11 gezeigten Ausgangsposition relativ zu dem Insertergehäuse 20 hält. In dem Halteeingriff hintergreift die Haltefeder eine an dem Griff 10, 12 geformte Haltestruktur 17, die in den Figuren 1, 2, 3 und 5 erkennbar ist. Der Halteeingriff ist gegen die rückstellende Elastizitätskraft der Haltefeder lösbar.

Der Antrieb umfasst ein Vortriebselement 22, das in und gegen eine Vortriebsrichtung V linear beweglich in dem Insertergehäuse 20 angeordnet ist. Die Vortriebsrichtung V fällt mit einer zentralen Längsachse des Insertergehäuses 20 zusammen. Der Antrieb umfasst ferner einen Krafterzeuger 23, der in die Vortriebsrichtung V auf das Vortriebselement 22 wirkt. Der Krafterzeuger 23 umfasst zwei Paare von gelenkig miteinander verbundenen Schenkeln 24, wobei die beiden Paare von Schenkeln 24 symmetrisch bezüglich der zentralen Längsachse, d. h. symmetrisch zur Vortriebsrichtung V, des Insertergehäuses 20 angeordnet sind. Jedes der Schenkelpaare ist in einem zum Insertergehäuse 20 ortsfesten Drehgelenk 25 aufgehängt. Die beiden Schenkel 24 des jeweiligen Schenkelpaars sind in einem freien Drehgelenk 26 miteinander drehbeweglich verbunden. Ferner ist der von dem ortsfesten Gelenk 25 abgewandte Schenkel 24 jeweils in einem Drehgelenk 27 mit dem Vortriebselement 22 verbunden. Nicht dargestellte Federn oder gegebenenfalls auch nur eine Feder spannt diese Schenkel-Gelenk-Vortriebselement-Anordnung in die Vortriebsrichtung V. Die Anordnung aus Schenkeln 24 und Gelenken 25, 26 und 27 führt das Vortriebselement 22; zusätzlich oder stattdessen könnte die Mantelinnenfläche des Insertionsgehäuses 23 das Vortriebselement 22 führen. Ferner ist ein Blockierglied 29 vorgesehen, das mit dem Insertergehäuse 20 in einem Blockiereingriff ist, der eine Vortriebsbewegung des Vortriebselements 22 verhindert. Das Blockierglied 29 kann den Blockiereingriff mit der von dem Insertergehäuse 20 gebildeten Hüllstruktur oder ebenso mit einer damit in Bezug auf die Vortriebsrichtung V fest verbundenen sonstigen Struktur bilden. Der Blockiereingriff ist durch Betätigung eines druckknopfartigen Auslösers 28 lösbar.

Der Inserter umfasst ferner ein Aktivierungsglied 21, das mit dem Insertergehäuse 20 in und gegen die Vortriebsrichtung V beweglich verbunden ist. Das Aktivierungsglied 21 bildet in Bezug auf das Insertergehäuse 20 eine Muffe, so dass insgesamt ein zweiteiliges, teleskopierbares Insertergehäuse mit Gehäuseteilen 20 und 21 erhalten wird. Der Unterscheidung in funktionaler Hinsicht wegen wird das Gehäuseteil 21 jedoch weiterhin als Aktivierungsglied bezeichnet. Das Aktivierungsglied 21 bildet die Unterseite U₂₁ des Inserters, mit welcher der Inserter für die Platzierung des Insertionskopfs auf der Gewebeoberfläche aufsetzbar ist und vorzugsweise auch aufgesetzt wird. In der in Figur 11 vom Insertionskopf eingenommenen Ausgangsposition weisen die Unterseite U₂₁ des Inserters und die Unterseite U des gehaltenen Insertionskopfs jeweils in die Vortriebsrichtung V, die für die beiden Unterseiten zumindest im Wesentlichen eine Flächennormale bildet.

Das Aktivierungsglied 21 umfasst ein äußeres Hülsenteil und ein inneres Hülsenteil, die an der Unterseite U₂₁ miteinander verbunden sind und zwischen sich einen Ringspalt freilassen. Das Insertergehäuse 20 ragt in diesen Ringspalt und führt das Aktivierungsglied 21 gleitbeweglich.

In dem in Figur 11 gezeigten Zustand nimmt das Aktivierungsglied 21 relativ zu dem Insertergehäuse 20 eine eingefahrene Position ein und der Inserter weist eine in Vortriebsrichtung V gemessen kürzeste Länge auf. In diesem Zustand des Inserters wird der Insertionskopf eingesetzt, d. h. in den Halteeingriff mit der Halteeinrichtung des Inserters gebracht. Anstatt den Insertionskopf einzusetzen, kann der Inserter auch über den auf einer Auflage liegenden Insertionskopf gestülpt werden. Die Position und Geometrie der Halteeinrichtung ist so gewählt, dass der Halteeingriff bei dem Aufstülpen automatisch entsteht. Unmittelbar nach dem Aufnehmen des Insertionskopfs, beispielsweise durch Einsetzen, befindet sich die Einführeinrichtung 5 des Insertionskopfs in ihrer Schutzposition. In diesem Sinne ist der Insertionskopf noch inaktiv. Der Inserter ist mit Mitteln ausgestattet, nämlich dem Aktivierungsglied 21, durch deren Betätigung die Einführeinrichtung in die Einführposition bewegt und auf diese Weise der Insertionskopf aktiviert werden kann.

Für die Aktivierung bilden das Aktivierungsglied 21 und der Insertionskopf miteinander ein Gelenk, im Ausführungsbeispiel ein Kurvengelenk. Die beiden Gelenkelemente des Gelenks sind eine Führungskurve 21 a, die das Aktivierungsglied 21 bildet, und ein von der beweglichen Griffkomponente 12 gebildetes Eingriffselement 12a. In der Kopplung, über die das Aktivierungsglied 21 auf die Einführeinrichtung 5 einwirkt, bildet die bewegliche Griffkomponente 12 ein Eingangs- bzw. Empfangsglied des Insertionskopfs. Wird das Aktivierungsglied 21 relativ zu dem Insertergehäuse 20 in die Vortriebsrichtung V bewegt, gleitet die Führungskurve 21a über das Eingriffselement 12a, d. h. über die das Eingriffselement 12a bildende Kontaktfläche des Empfangsglieds, d. h. der beweglichen Griffkomponente 12. Durch den Druckkontakt und den zur Vortriebsrichtung V geneigten Verlauf der Führungskurve 21a bewegt sich die Griffkomponente 12 quer zu der Vortriebsrichtung V auf die andere Griffkomponente 10 zu, und die Einführeinrichtung 5 schwenkt wie zum Insertionskopf als solchem beschrieben in die Einführposition. Die bewegliche Griffkomponente 12 bildet das Eingriffselement 12a an ihrem von der Basis 1, 2 abgewandten oberen Ende, im Ausführungsbeispiel mit ihrer äußeren Kante. Die Führungskurve 21a ist der Unterseite U₂₁ des Inserters zugewandt. Die Neigung ist so gewählt, dass sich die Führungskurve 21 a von einem von der Unterseite U₂₁ abgewandten Ende in Vortriebsrichtung V von dem Insertionskopf bzw. der im aktivierten Zustand ausgeschwenkten Einführeinrichtung 5 oder der zentralen Längsachse des Inserters weg neigt. Der Neigungswinkel ist überall konstant, die Führungskurve 21 a ist eine Schräge, d. h. eine schräge Linie oder Fläche.

Für die praktische Handhabung bietet es sich an, dass der Benutzer den Inserter nach dem Aufnehmen des Insertionskopfs mit der einen Hand am Aktivierungsglied 21 hält, beispielsweise indem er das Aktivierungsglied 21 umgreift, und mit der anderen Hand das Insertergehäuse 20 relativ zu dem gehaltenen Aktivierungsglied 21 gegen die Vortriebsrichtung V zieht. Auch dies wird als Betätigung des Aktivierungsglieds verstanden. Das Vortriebselement 22 und der Krafterzeuger 23 bewegen sich gemeinsam mit dem Insertergehäuse 20 relativ zu dem Aktivierungsglied 21. Der von der Halteeinrichtung in der Ausgangsposition gehaltene Insertionskopf wird mitgenommen, d. h. bewegt sich gleichermaßen relativ zu dem Aktivierungsglied 21 entgegen der Vortriebsrichtung V. Das Eingriffselement 12a gleitet längs der Führungskurve 21 a. Über diese auf reinem Druckkontakt beruhende Schnittstelle wird die bewegliche Griffkomponente 12 quer zu der Vortriebsrichtung V bewegt, und die Einführeinrichtung 5 schwenkt in die Einführposition. Der Insertionskopf ist am Ende der Ausfahrbewegung, die das Insertionsgehäuse 20 und das Aktivierungsglied 21 relativ zueinander ausführen, aktiviert.

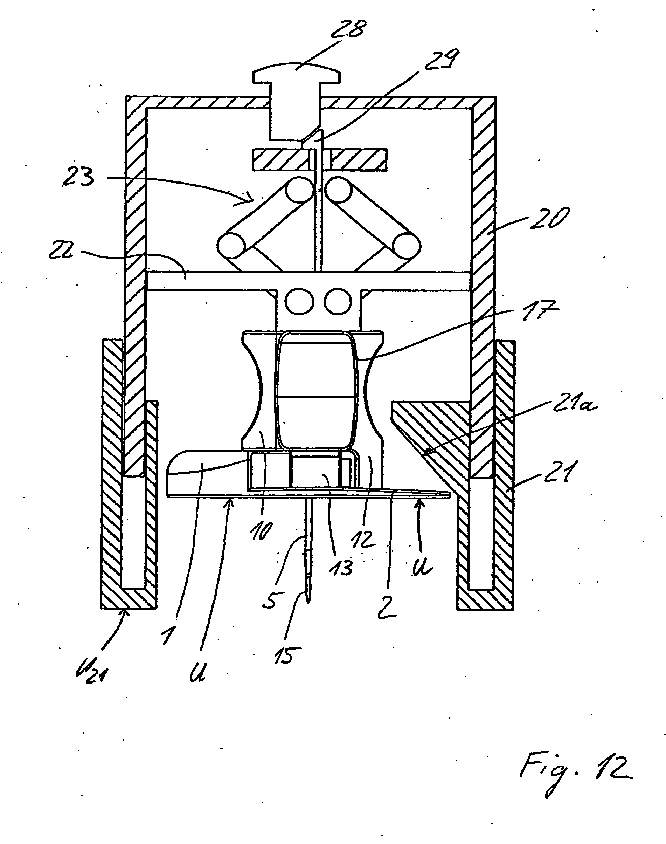

Figur 12 zeigt das System aus Inserter und Insertionskopf in dessen aktiviertem Zustand. Das Insertergehäuse 20 und das Aktivierungsglied 21 nehmen relativ zueinander die ausgefahrene Position ein. In dem ausgefahrenen Zustand umgeben die Wände des Insertergehäuses 20 und des Aktivierungsglieds 21 den aktivierten Insertionskopf bis über das freie Ende der Einführeinrichtung 5 und der Einstecheinrichtung 15 hinaus, d. h. die Spitze der Einstecheinrichtung 15 steht ein kleines Stück weit hinter der Unterseite U₂₁ des Inserters zurück.

Das Insertergehäuse 20 und das Aktivierungsglied 21 sind in der ausgefahrenen Position relativ zueinander blockiert. Relativbewegungen in oder gegen die Vortriebsrichtung V sind in dem blockierten Zustand nicht möglich. Bei Erreichen der ausgefahrenen Position blockieren sich das Insertergehäuse 20 und das Aktivierungsglied 21 aneinander automatisch.

Für die Platzierung des Insertionskopfs setzt der Benutzer den Inserter auf der Hautoberfläche auf. Bei aufgesetztem Inserter drückt der Benutzer auf den Auslöser 28. Der Auslöser 28 wirkt über ein Kurvengelenk, im Ausführungsbeispiel ein einfaches Paar von Schrägen, auf das Blockierglied 29. Unter der Einwirkung des Auslösers 28 bewegt sich das Blockierglied 29 aus dem Blockiereingriff mit dem Insertergehäuse 20, so dass sich das Vortriebselement 22 unter der Einwirkung des Krafterzeugers 23 in die Vortriebsrichtung V bewegen kann. Der Krafterzeuger 23 beschleunigt das Vortriebselement 22 schlagartig. Das Vortriebselement 22 wirkt auf den Insertionskopf wie ein Hammer. Im ersten Abschnitt der Vortriebsbewegung federt die Haltefeder aus dem Halteeingriff mit der Haltestruktur 17 des Insertionskopfs, d. h. der Halteeingriff löst sich. Die Beschleunigung des Vortriebselements 22 in die Vortriebsrichtung V ist so groß, dass der reine Druckkontakt zwischen dem Vortriebselement 22 und dem Insertionskopf sicher erhalten bleibt, bis die Unterseite U des Insertionskopfs auf der gleichen Höhe wie die Unterseite U₂₁ des Inserters und somit auf der Gewebeoberfläche platziert ist. Bereits zuvor durchdringt die Einstecheinrichtung 15 die Hautoberfläche, dringt in das Gewebe ein und nimmt dabei die Einführeinrichtung 5 mit.

Nachdem der Insertionskopf auf der Hautoberfläche platziert ist, greift der Benutzer den Griff 10, 12 und zieht ihn von der Basis 1, 2 ab. Dabei wird die Einstecheinrichtung 15 automatisch aus der Einführeinrichtung 5 heraus- und von der Basis 1, 2 abgezogen.

Um auch das Herausziehen der Einstecheinrichtung 15 zu automatisieren, bleibt der Halteeingriff zwischen der Halteeinrichtung des Inserters und der Haltestruktur 17 des Insertionskopfs in einer vorteilhaften Modifikation des Inserters bestehen und wird nicht, wie im beschriebenen Ausführungsbeispiel, durch die Beschleunigung des Vortriebselements 22 gelöst. In einer derartigen Modifizierung kann die Halteeinrichtung insbesondere ortsfest mit dem Vortriebselement 22 verbunden sein, so dass sie dessen Ausstoßbewegung in die Vortriebsrichtung V mitmacht. Um den Halteeingriff zu lösen, kann der Inserter mit einem Abstreifer ausgestattet sein, der nach dem Wegnehmen des Inserters vom Gewebe bei einem Zusammenschieben von Insertergehäuse 20 und Aktivierungsglied 21 automatisch den Insertionskopf aus dem Halteeingriff löst. Alternativ kann solch ein Abstreifer auch vollkommen unabhängig vom Aktivierungsglied 21 vorgesehen und separat betätigbar sein, um den Halteeingriff zu lösen.

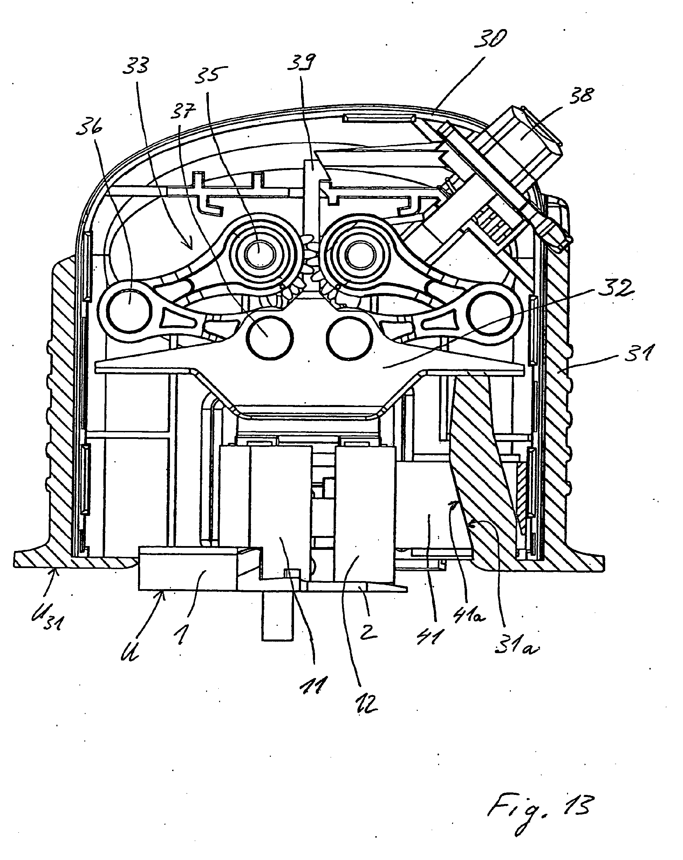

Die Figuren 13 bis 15 zeigen ein aus einem Insertionskopf und einem Inserter bestehendes System eines zweiten Ausführungsbeispiels. Der Insertionskopf ist derjenige der Figuren 7 bis 10, kann aber auch der gleiche wie im ersten Ausführungsbeispiel sein. Nur bei dem Inserter handelt es sich um eine Modifikation. Diejenigen Komponenten des Inserters des zweiten Ausführungsbeispiels, die in Bezug auf ihre Funktion mit den Komponenten des Inserters des ersten Ausführungsbeispiels vergleichbar sind, werden jeweils mit den um die Zahl zehn erhöhten Bezugszeichen des ersten Ausführungsbeispiels belegt. So gelten insbesondere zum Insertergehäuse 30 und dem Aktivierungsglied 31, was deren Form und Verbindung sowie Relativbeweglichkeit anbetrifft, die Ausführungen zum ersten Ausführungsbeispiel. Grundsätzlich das Gleiche gilt auch in Bezug auf das Vortriebselement 32, die Halteeinrichtung und der Krafterzeuger 33 sowie den Auslöser 38 und das Blockierglied 39. Soweit im Folgenden auf Unterschiede nicht hingewiesen wird und sich auch aus den Figuren nichts anderes ergibt, gelten die Ausführungen zum ersten Ausführungsbeispiel gleichermaßen auch für das zweite Ausführungsbeispiel.

Der Inserter des zweiten Ausführungsbeispiels unterscheidet sich von dem Inserter des ersten Ausführungsbeispiels im Wesentlichen im Hinblick auf das Gelenk, über welches das Aktivierungsglied 31 auf den Insertionskopf wirkt, um diesen durch die Aufziehbewegung des Insertergehäuses 30 relativ zu dem Aktivierungsglied 31 zu aktivieren. Im zweiten Ausführungsbeispiel bildet der Inserter selbst das Gelenk, nämlich mit zwei Gelenkelementen 31a und 41 a, von denen eines das Aktivierungsglied 31 und das andere ein Effektorglied 41 bildet. Das Effektorglied 41 wird quer zu der Vortriebsrichtung V, im Ausführungsbeispiel rechtwinklig zu der Vortriebsrichtung V, hin und her beweglich von dem Insertionsgehäuse 30 gelagert. Das Gelenk 31 a, 41a ist wieder ein Kurvengelenk.

Die Führungskurve 31 a entspricht der Führungskurve 21 a des ersten Ausführungsbeispiels. Das Effektorglied 41 bildet das Eingriffselement 41a, das bei Verlängerung des Inserters an der Führungskurve 31 a entlang gleitet und aufgrund des geneigten Verlaufs der Führungskurve 31 a bei dem Aufziehen des Inserters eine Querbewegung des Effektorglieds 41 in Richtung auf die zentrale Längsachse des Inserters bewirkt. In dem Gelenk 31a, 41a wird somit die gegen die Vortriebsrichtung V weisende Bewegung, die das Insertergehäuse 30 bei dem Aufziehen relativ zu dem Aktivierungsglied 31 ausführt, in die Querbewegung des Effektorglieds 41 umgewandelt. Dessen Gelenkelement bzw. Eingriffselement 41a ist selbst in der Art einer Führungskurve gebildet, wird hier getriebetechnisch jedoch als Eingriffselement bezeichnet. Das Eingriffselement 41a könnte alternativ auch als beispielsweise einfacher Nocken oder Noppen geformt sein. Ebenso könnte das Eingriffselement 41 a als Führungskurve bezeichnet und in einer anderen Modifikation das Gelenkelement 31a als vorragender Nocken oder Noppen geformt werden.

Die Schnittstelle, über welche der Inserter den Insertionskopf aktiviert, ist wieder als reiner Druckkontakt gebildet und besteht zwischen dem Effektorglied 41 und dem Empfangsglied bzw. der beweglichen Griffkomponente 12 des Insertionskopfs. Dieser reine, man könnte auch sagen lose Druckkontakt vereinfacht die Handhabung, da für die Aktivierung keine besondere Gelenkverbindung hergestellt werden muss, es genügt das Aufnehmen des Insertionskopfs in Kombination mit der Betätigung des Aktivierungsglieds 31, was in den Ausführungsbeispielen durch die Aufziehbewegung erfolgt. Der Druckkontakt, d. h. die von dem Effektorglied 41 ausgeübte Druckkraft, wirkt auf die bewegliche Griffkomponente 12 parallel zu der Richtung ihrer Beweglichkeit relativ zu der Basis 1, 2. Durch Zwischenschaltung des Effektorglieds 41 und Verlagerung des Gelenks 31 a, 41 a gänzlich zum Inserter wird auf die Griffkomponente 12 im zweiten Ausführungsbeispiel vorteilhafterweise quer zu der Richtung der Beweglichkeit der Griffkomponente 12 keine Kraft ausgeübt.

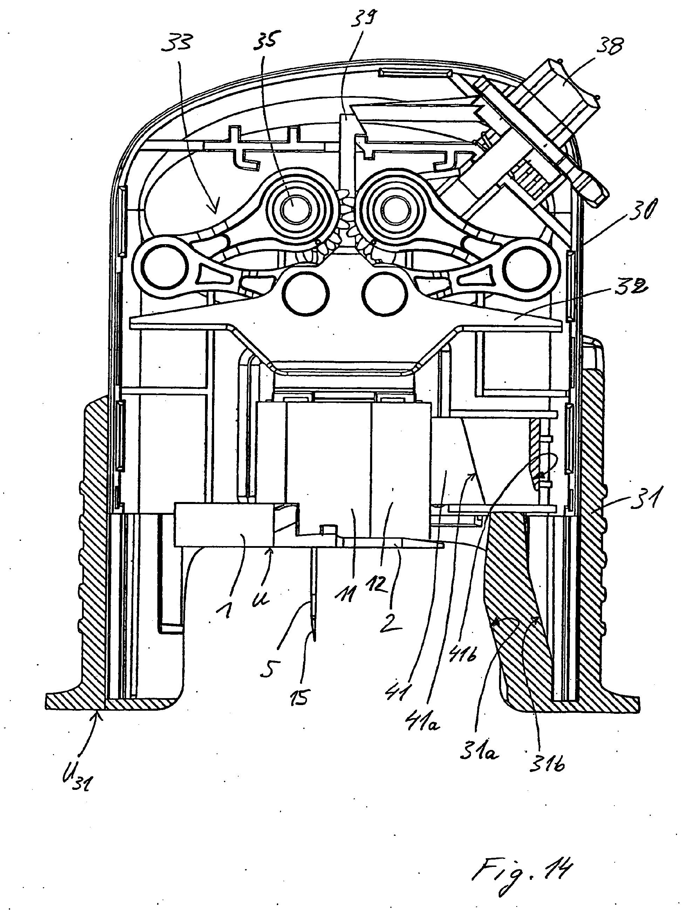

Figur 14 zeigt das System mit aktiviertem Insertionskopf. Im Verlaufe der Aufziehbewegung des Insertergehäuses 30, was auch als Betätigung des Aktivierungsglieds 31 verstanden wird, wurden die Einführeinrichtung 5 und die Einstecheinrichtung 15 in die Einführposition geschwenkt, so dass ihre gemeinsame Längsachse in die Vortriebsrichtung V weist. Die bewegliche Griffkomponente 12 hat wie zum Insertionskopf beschrieben die Verbindung zwischen dem Griff 10, 12 und der Basis 1, 2 gelöst. Der zwischen der Einführeinrichtung 5 und der Einstecheinrichtung 15 bestehende Reibschluss hält jedoch die Basis 1, 2 wie im ersten Ausführungsbeispiel an dem im Halteeingriff befindlichen Griff 10, 12.

Durch Betätigung des Auslösers 38 wird der Blockiereingriff, in dem sich das Blockierglied 39 noch mit dem Insertergehäuse 30 oder einer damit fest verbundenen Struktur befindet, gelöst und der Krafterzeuger 33 beschleunigt das Vortriebselement 32 in die Vortriebsrichtung V. Die Beschleunigung erfolgt wieder schlagartig, so dass auch die Antriebseinrichtung 32, 33 des zweiten Ausführungsbeispiels in der Art eines Hammers wirkt. Die Antriebskraft wird von zwei Schenkelfedern erzeugt, von denen je eine auf eines der beiden Schenkelpaare wirkt. Die im ortsfesten Drehlager 35 befestigten Schenkel 24 sind über einen Zahneingriff miteinander gekoppelt, der für eine synchrone Ausfahrbewegung der beiden Schenkelpaare sorgt.

Um den Inserter nach Platzierung des Insertionskopfs für eine erneute Verwendung bereitmachen zu können, muss das Effektorglied 41 aus der in Figur 14 gezeigten Endposition wieder zurück in die in Figur 13 gezeigte Endposition bewegt werden. Für diese Rückholbewegung bilden das Aktivierungsglied 31 und das Effektorglied 41 ein weiteres Gelenk 31b, 41b, das im Ausführungsbeispiel ebenfalls ein Kurvengelenk ist. Das Aktivierungsglied 31 bildet für das weitere Gelenk die Führungskurve 31b, und das Effektorglied 41 bildet das Eingriffselement 41b. Die Führungskurve 31b verläuft zumindest im Wesentlichen parallel zu der Führungskurve 31 a. Die Führungskurven 31 a und 31b sind an dem inneren Hülsenteil des Aktivierungsglieds 31 gebildet, die Führungskurve 31 a an der Innenfläche und die Führungskurve 31 b an der Außenfläche des inneren Hülsenteils. Sie liegen sich in etwa auf der gleichen Höhe, bezogen auf die Vortriebsrichtung V, gegenüber. Das Eingriffselement 41 b liegt dem Eingriffselement 41 a ebenfalls gegenüber mit einem lichten Abstand, so dass das innere Hülsenteil des Aktivierungsglieds 31 zwischen den beiden Eingriffselementen 41 a und 41b ein- und ausfahren kann.

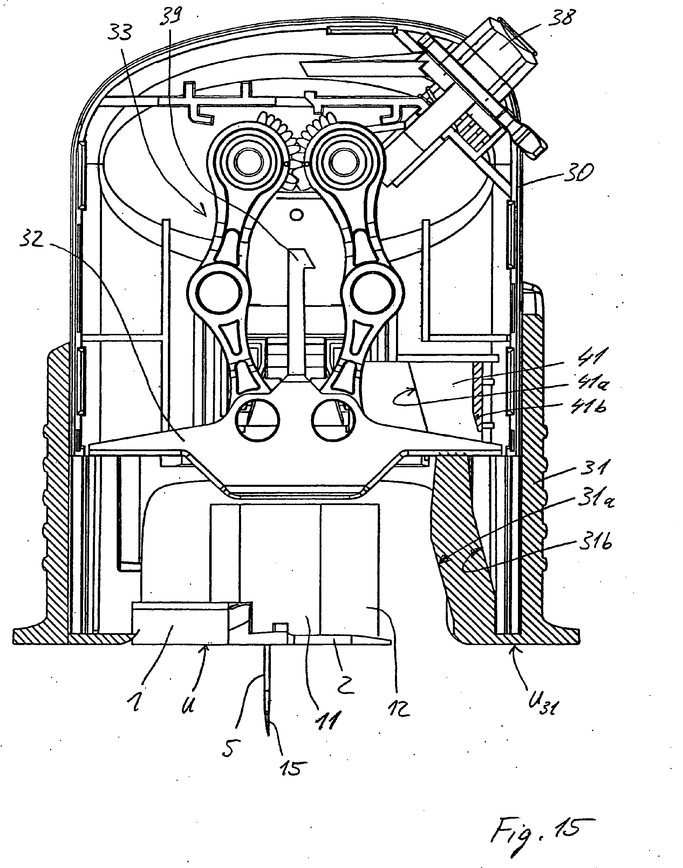

Figur 15 zeigt das System des zweiten Ausführungsbeispiels mit dem platzierten Insertionskopf. Der Inserter wird von dem Insertionskopf entfernt. Anschließend zieht der Benutzer den Griff 10, 12 von der Basis 1, 2 ab und schließt den Insertionskopf an einen Katheter einer Infusionspumpe an. In einer auch bereits zum ersten Ausführungsbeispiel erwähnten Modifikation, in welcher die Halteeinrichtung ortsfest mit dem Vortriebselement 32 verbunden ist und demgemäß den Griff 10, 12 noch halten kann, werden der Inserter und damit gemeinsam der noch gehaltene Griff 10, 12 von der Basis entfernt. Anschließend wird vorzugsweise mittels eines zusätzlichen Abstreifers der Halteeingriff gelöst und der Griff 10, 12 mit der Einstecheinrichtung 15 oder nur dieser alleine entsorgt.

Um den Inserter für die Verwendung mit einem weiteren Insertionskopf vorzubereiten, schiebt der Benutzer das Insertergehäuse 30 und das Aktivierungsglied 31 wieder zusammen in die eingefahrene Position, wie sie mit eingesetztem Insertionskopf in Figur 13 dargestellt ist. Bei der Einfahrbewegung fährt das innere Hülsenteil des Aktivierungsglieds 31 zwischen die Eingriffselemente 41a und 41b des Effektorglieds 41. Bei dieser Einfahrbewegung entsteht die weitere Gelenkverbindung zwischen der Führungskurve 31b und dem Eingriffselement 41b. Bei der Einfahrbewegung wird somit in dem Gelenk 31b, 41b das Effektorglied 41 wieder in die in Figur 13 eingenommene Endposition zurückbewegt, d. h. in Bezug auf die zentrale Längsachse des Inserters quer, vorzugsweise radial, nach auswärts bewegt.

Das unter der Einwirkung der Federeinrichtung 33 in die Vortriebsrichtung V ausgefahrene Vortriebselement 32 liegt einer Stirnseite des inneren Hülsenteils, die von der Unterseite U₃₁ des Aktivierungsglieds 31 abgewandt ist, gegenüber. Durch Anschlagkontakt gegen diese Stirnseite wird die Vortriebsbewegung des Vortriebselements 32 gestoppt. Das Aktivierungsglied 31 ist geometrisch so bemessen, dass es in der ausgefahrenen Position des Teleskops 30, 31 das Vortriebselement 32 genau dann stoppt, wenn die Unterseite U des Insertionskopfs die Höhe der Unterseite U₃₁ erreicht hat und daher bei aufgesetztem Inserter die Hautoberfläche gerade kontaktiert. Bei der Einfahrbewegung des Insertergehäuses 30 relativ zum Aktivierungsglied 31 bzw. des Aktivierungsglieds 31 relativ zu dem Insertergehäuse 30 wird das Vortriebselement 32 wegen des Anschlagkontakts vom Aktivierungsglied 31 gegen die Kraft des Krafterzeugers 33 tiefer in das Insertergehäuse 30 gedrückt, bis das Blockierglied 39 wieder im Blockiereingriff ist, wie ihn beispielhaft die Figuren 13 und 14 zeigen.

### Bezugszeichen:

- 1: Basis, Aufnahme
- 2: Basis, Flachteil
- 3: Ausnehmung
- 4: Ausnehmung
- 5: Einführeinrichtung
- 6: Gelenkelement
- 7: Zuführung
- 8: Zahnrad
- 9: Verbindungselement
- 10: Erste Griffkomponente
- 11: Erste Griffkomponente
- 12: Zweite Griffkomponente, Empfangsglied
- 12a: Gelenkelement, Eingriffselement, Druckkontaktfläche
- 13: Lasche
- 14: Aufnahme
- 15: Einstecheinrichtung
- 16: Verbindungselement
- 17: Haltestruktur
- 18: Zahnstange
- 19: Verbindungselement
- 20: Insertergehäuse
- 21: Aktivierungsglied
- 21 a: Gelenkelement, Führungskurve
- 22: Vortriebselement
- 23: Krafterzeuger
- 24: Schenkel
- 25: Drehgelenk
- 26: Drehgelenk
- 27: Drehgelenk
- 28: Auslöser

- 29: Blockierglied
- 30: Insertergehäuse
- 31: Aktivierungsglied
- 31 a: Gelenkelement, Führungskurve
- 31 b: Gelenkelement, Führungskurve
- 32: Vortriebselement
- 33: Krafterzeuger
- 34: Schenkel
- 35: Drehgelenk
- 36: Drehgelenk
- 37: Drehgelenk
- 38: Auslöser
- 39: Blockierglied
- 40: -
- 41: Effektorglied
- 41 a: Gelenkelement, Eingriffselement
- 41 b: Gelenkelement, Eingriffselement
- U: Unterseite
- V: Vortriebsrichtung

## Patentansprüche

1. Insertionskopf für medizinische oder pharmazeutische Anwendungen, der Insertionskopf umfassend:
a) eine Basis mit einer auf organischem Gewebe platzierbaren Unterseite (U), wobei die Basis einen Aufnahmeteil (1) und einen Flachteil (2) aufweist, die in einem Stück geformt sind,
b) eine in das Gewebe einführbare, von der Basis (1, 2) beweglich gelagerte Einführeinrichung (5),
c) wobei die Einführeinrichtung (5) relativ zu der Basis (1, 2) aus einer Schutzposition, in der ein freies Ende der Einführeinrichtung (5) hinter der Unterseite (U) der Basis (1, 2) zurücksteht, in eine Einführposition bewegbar ist, in der das freie Ende der Einführeinrichtung (5) über die Unterseite (U) der Basis (1, 2) vorragt,
d) und eine zu der Unterseite (U) der Basis (1, 2) offene und von der Basis (1, 2) lösbare Aufnahme (14), die zumindest das freie Ende der Einführeinrichtung (5) in deren Schutzposition aufnimmt, wobei
e) ein Griff (11, 12, 14) mit der Basis (1, 2) lösbar verbunden ist und die lösbare Aufnahme (14) bildet,
wobei
f) der Griff (11, 12, 14) einen von einer Oberseite der Basis (1, 2) aufragenden Griffteil (11, 12) aufweist und die lösbare Aufnahme (14) von dem Griffteil (11, 12) seitlich abragt, wobei die lösbare Aufnahme (14) die Basis (1, 2) topf- oder schüsselartig umgibt, **dadurch gekennzeichnet, dass** die Basis (1, 2) und ein Gelenkelement (6) miteinander ein Gelenk bilden und die Einführeinrichtung (5) von dem Gelenkelement (6) abragt, vorzugsweise indem eine Längsrichtung der Einführeinrichtung (5) eine Bewegungsachse des Gelenks schneidet oder kreuzt.

2. Insertionskopf nach Anspruch 1, **dadurch gekennzeichnet, dass** die Basis (1, 2) eine zu der Unterseite (U) der Basis (1, 2) offene weitere Aufnahme (1) bildet, die einen Abschnitt der Einführeinrichtung (5) in deren Einführposition aufnimmt und dass die Einführeinrichtung (5) durch eine Ausnehmung (4) der weiteren Aufnahme (1) in die lösbare Aufnahme (14) ragt.

3. Insertionskopf nach dem vorhergehenden Anspruch, **dadurch gekennzeichnet, dass** die lösbare Aufnahme (14) die weitere Aufnahme (1) in Längsrichtung der Einführeinrichtung (5) verlängert, vorzugsweise die weitere Aufnahme (1) aufnimmt.

4. Insertionskopf nach einem der zwei vorhergehenden Ansprüche, **dadurch gekennzeichnet, dass** die weitere Aufnahme (1) eine äußere Oberfläche aufweist, die von ihrer Oberseite in Richtung auf die Unterseite (U) der Basis (1, 2) nach außen rund vorgewölbt abfällt.

5. Insertionskopf nach einem der vorhergehenden Ansprüche, **dadurch gekennzeichnet, dass** die Einführeinrichtung (5) in die Einführposition schwenkbar ist.

6. Insertionskopf nach einem der vorhergehenden Ansprüche, **dadurch gekennzeichnet, dass** der Insertionskopf einen Griff (11, 12, 14) und der Griff (11, 12, 14) eine relativ zu der Basis (1, 2) bewegliche Griffkomponente (12) umfasst oder als solche gebildet ist und die bewegliche Griffkomponente (12) mittels eines Getriebes (8, 18) mit der Einführeinrichtung (5) gekoppelt ist, dass eine relativ zu der Basis (1, 2) stattfindende Bewegung der beweglichen Griffkomponente (12) die Bewegung der Einführeinrichtung (5) bewirkt.

7. Insertionskopf nach dem vorhergehenden Anspruch, **dadurch gekennzeichnet, dass** das Getriebe (8, 18) die bewegliche Griffkomponente (12) mittels Zahneingriffs, vorzugsweise eines Zahnrads (8) und einer Zahnstange (18), mit der Einführeinrichtung (5) koppelt.

8. Insertionskopf nach dem vorhergehenden Anspruch, **dadurch gekennzeichnet, dass** die bewegliche Griffkomponente (12) mit einer Zahnstange (18), die vorzugsweise an der beweglichen Griffkomponente (12) geformt ist, und die Einführeinrichtung (5) mit einem mit der Zahnstange (18) in dem Zahneingriff befindlichen Zahnrad (8) verbunden ist.

9. Insertionskopf nach einem der zwei vorhergehenden Ansprüche, **dadurch gekennzeichnet, dass** die Einführeinrichtung (5) um eine Rotationsachse schwenkbar und drehsteif mit einem um die Rotationsachse drehbaren, in dem Zahneingriff befindlichen Zahnrad (8) verbunden ist.

10. Insertionskopf nach einem der Ansprüche 1 bis 9, **dadurch gekennzeichnet, dass** der Griff (11, 12, 14) eine relativ zu der Basis (1, 2) bewegliche Griffkomponente (12) aufweist und eine zwischen der Basis (1, 2) und dem Griff (11, 12) bestehende Verbindung sich bei einer Bewegung der beweglichen Griffkomponente (12) löst.

11. Insertionskopf nach dem vorhergehenden Anspruch, **dadurch gekennzeichnet, dass** an der Basis (1, 2) ein erstes Verbindungselement (9) und an dem Griff (11, 12, 14) ein zweites Verbindungselement (19) geformt ist, dass die Verbindungselemente (9, 19) miteinander in einem die Verbindung schaffenden Eingriff sind und dass wenigstens eines der Verbindungselemente (9, 19) vorzugsweise gegen eine Elastizitätskraft aus dem Eingriff bewegbar ist.

12. Insertionskopf nach einem der zwei vorhergehenden Ansprüche, **dadurch gekennzeichnet, dass** ein erstes Verbindungselement (9) und ein zweites Verbindungselement (19) miteinander in einem die Verbindung schaffenden Eingriff sind und die bewegliche Griffkomponente (12) bei ihrer Bewegung eines der Verbindungselemente (9, 19) kontaktiert und vorzugsweise gegen eine Elastizitätskraft aus dem Eingriff bewegt.

13. Insertionskopf nach einem der Ansprüche 6 bis 12, **dadurch gekennzeichnet, dass** der Griff (11, 12, 14) eine weitere Griffkomponente (11, 14) umfasst und die bewegliche Griffkomponente (12) und die weitere Griffkomponente (11, 14) zwischen zwei Fingern einer Hand greifbar sind, wobei mit je einem der Finger gegen eine der Griffkomponenten (11, 12, 14) drückend die bewegliche Griffkomponente (12) gegen die weitere Griffkomponente (11, 14) bewegbar ist.

14. Insertionskopf nach einem der Ansprüche 6 bis 13, **dadurch gekennzeichnet, dass** die bewegliche Griffkomponente (12) relativ zu der Basis (1, 2) verschiebbar ist.

15. Insertionskopf nach einem der Ansprüche 6 bis 14, **dadurch gekennzeichnet, dass** die bewegliche Griffkomponente (12) linear geführt ist.

16. Insertionskopf nach einem der Ansprüche 6 bis 15, **dadurch gekennzeichnet, dass** die bewegliche Griffkomponente (12) zumindest im Wesentlichen parallel zu der Unterseite (U) der Basis (1, 2) beweglich ist.

17. Insertionskopf nach einem der Ansprüche 6 bis 16, **dadurch gekennzeichnet, dass** der Griff (11, 12, 14) eine weitere Griffkomponente (11, 14) umfasst, die relativ zu der Basis (1, 2) im verbundenen Zustand unbeweglich ist und die lösbare Aufnahme (14) bildet.

18. Insertionskopf nach einem der Ansprüche 1 bis 17, **dadurch gekennzeichnet, dass** der Griff (11, 12, 14) von einer Oberseite der Basis (1, 2) aufragt.

19. Insertionskopf nach einem der vorhergehenden Ansprüche, **dadurch gekennzeichnet, dass** die Einführeinrichtung (5) flexibel ist und von einer Einstecheinrichtung (15) stabilisiert wird, die gemeinsam mit der Einführeinrichtung (5) aus der Schutzposition in die Einführposition bewegbar ist.

20. Insertionskopf nach dem vorhergehenden Anspruch, **dadurch gekennzeichnet, dass** die Einstecheinrichtung (15) sich bei der Bewegung der Einführeinrichtung (5) in die Einführposition mit der lösbaren Aufnahme (14) verbindet.

21. Insertionskopf nach einem der zwei vorhergehenden Ansprüche, **dadurch gekennzeichnet, dass** die Einstecheinrichtung (15) mit der lösbaren Aufnahme (14) von der Basis (1, 2) und der Einführeinrichtung (5) lösbar ist.

22. Insertionskopf nach einem der vorhergehenden Ansprüche, **dadurch gekennzeichnet, dass** die Einführeinrichtung (5) in eine Längsrichtung langgestreckt ist und die Längsrichtung mit der Unterseite (U) der Basis (1, 2) einen spitzen Winkel von weniger als 50° einschließt, wenn das freie Ende der Einführeinrichtung (5) bei der Bewegung in die Einführposition über die Unterseite (U) vortritt.

23. Insertionskopf nach einem der vorhergehenden Ansprüche, **dadurch gekennzeichnet, dass** die Basis (1, 2) die Einführeinrichtung (5) um eine Rotationsachse schwenkbar lagert und die Einfiihreinrichtung (5) eine Längsachse aufweist, welche die Rotationsachse schneidet oder in einem Abstand von höchstens der halben Länge der Einführeinrichtung (5) kreuzt.

## Claims

1. An insertion head for medical or pharmaceutical applications, the insertion head comprising:
a) a base having an underside (U) which can be placed on organic tissue, wherein the base has a receiving portion (1) and a flat portion (2) which are shaped in one piece,
b) an introduction device (5) which can be introduced into the tissue and which is supported movably by the base (1, 2),
c) wherein the introduction device (5) is movable relative to the base (1, 2) from a protection position in which a free end of the introduction device (5) is set back behind the underside (U) of the base (1, 2) into an introduction position in which the free end of the introduction device (5) projects beyond the underside (U) of the base (1, 2),
d) and a receiving means (14) which is open towards the underside (U) of the base (1, 2) and which is releasable from the base (1, 2) and which receives at least the free end of the introduction device (5) in the protection position thereof, wherein
e) a handle (11, 12, 14) is releasably connected to the base (1, 2) and forms the releasable receiving means (14), wherein
f) the handle (11, 12, 14) has a handle portion (11, 12) which projects up from a top side of the base (1, 2) and the releasable receiving means (14) projects laterally from the handle portion (11, 12), wherein the releasable receiving means (14) surrounds the base (1, 2) in a pot-like or tray-like configuration, **characterised in that** the base (1, 2) and a joint element (6) together form a joint and the introduction device (5) projects from the joint element (6), preferably by a longitudinal direction of the introduction device (5) intersecting or crossing an axis of movement of the joint.

2. An insertion head according to claim 1 **characterised in that** the base (1, 2) has a further receiving means (1) which is open to the underside (U) of the base (1, 2) and which receives a portion of the introduction device (5) in the introduction position thereof and the introduction device (5) projects through an opening (4) of the further receiving means (1) into the releasable receiving means (14).

3. An insertion head according to the preceding claim **characterised in that** the releasable receiving means (14) prolongs the further receiving means (1) in the longitudinal direction of the introduction device (5), and preferably receives the further receiving means (1).

4. An insertion head according to one of the two preceding claims **characterised in that** the further receiving means (1) has an outer surface which falls away outwardly in a round forwardly curved configuration from its top side in a direction towards the underside (U) of the base (1, 2).

5. An insertion head according to one of the preceding claims **characterised in that** the introduction device (5) is pivotable into introduction position.

6. An insertion head according to one of the preceding claims **characterised in that** the insertion head includes a handle (11, 12, 14) and the handle (11, 12, 14) includes a handle component (12) movable relative to the base (1, 2) or is formed as such and the movable handle component (12) is coupled to the introduction device (5) by means of a transmission (8, 18), such that a movement of the movable handle component (12), relative to the base (1, 2), causes the movement of the introduction device (5).

7. An insertion head according to the preceding claim **characterised in that** the transmission (8, 18) couples the movable handle component (12) to the introduction device (5) by means of tooth engagement, preferably a gear (8) and a rack (18).

8. An insertion head according to the preceding claim **characterised in that** the movable handle component (12) is connected to a rack (18) which is preferably formed on the movable handle component (12) and the introduction device (5) is connected to a gear (8) in tooth engagement with the rack (18).

9. An insertion head according to the two preceding claims **characterised in that** the introduction device (5) is connected pivotably about an axis of rotation and non-rotatably to a gear (8) which is rotatable about the axis of rotation and which is in the tooth engagement.

10. An insertion head according to one of claims 1 to 9 **characterised in that** the handle (11, 12, 14) has a handle component (12) movable relative to the base (1, 2) and a connection between the base (1, 2) and the handle (11, 12) is released upon a movement of the movable handle component (12).

11. An insertion head according to the preceding claim **characterised in that** a first connecting element (9) is formed at the base (1, 2) and a second connecting element (19) is formed at the handle (11, 12, 14), the connecting elements (9, 19) are in an engagement with each other that provides the connection, and at least one of the connecting elements (9, 19) is movable out of the condition of engagement preferably against an elasticity force.

12. An insertion head according to one of the two preceding claims **characterised in that** a first connecting element (9) and a second connecting element (19) are in an engagement with each other that provides the connection and the movable handle component (12) in the movement thereof contacts one of the connecting elements (9, 19) and moves it out of the condition of engagement preferably against an elasticity force.

13. An insertion head according to one of claims 6 to 12 **characterised in that** the handle (11, 12, 14) includes a further handle component (11, 14) and the movable handle component (12) and the further handle component (11, 14) can be gripped between two fingers of a hand, wherein the movable handle component (12) is movable towards the further handle component (11, 14) by pressing with a respective one of the fingers against one of the handle components (11, 12, 14).

14. An insertion head according to one of claims 6 to 13 **characterised in that** the movable handle component (12) is slidable relative to the base (1, 2).

15. An insertion head according to one of claims 6 to 14 **characterised in that** the movable handle component (12) is linearly guided.

16. An insertion head according to one of claims 6 to 15 **characterised in that** the movable handle component (12) is movable at least substantially parallel to the underside (U) of the base (1, 2).

17. An insertion head according to one of claims 6 to 16 **characterised in that** the handle (11, 12, 14) includes a further handle component (11, 14) which is immovable relative to the base (1, 2) in the connected condition and forms the releasable receiving means (14).

18. An insertion head according to one of claims 1 to 17 **characterised in that** the handle (11, 12, 14) projects up from a top side of the base (1, 2).

19. An insertion head according to one of the preceding claims **characterised in that** the introduction device (5) is flexible and is stabilised by an injection device (15) which is movable jointly with the introduction device (5) out of the protection position into the introduction position.

20. An insertion head according to the preceding claim **characterised in that** the injection device (15) is connected to the releasable receiving means (14) in the movement of the introduction device (5) into the introduction position.

21. An insertion head according to one of the two preceding claims **characterised in that** the injection device (15) is releasable with the releasable receiving means (14) from the base (1, 2) and the introduction device (5).

22. An insertion head according to one of the preceding claims **characterised in that** the introduction device (5) is elongate in a longitudinal direction and the longitudinal direction includes an acute angle of less than 50° with the underside (U) of the base (1, 2) when the free end of the introduction device (5) protrudes beyond the underside (U) in the movement into the introduction position.

23. An insertion head according to one of the preceding claims **characterised in that** the base (1, 2) mounts the introduction device (5) pivotably about axis of rotation and the introduction device (5) has a longitudinal axis which intersects the axis of rotation or crosses it at a spacing of at most half the length of the introduction device (5).

## Revendications

1. Tête d'injection pour des applications médicales ou pharmaceutiques, la tête d'injection comprenant :
a) une base dotée d'un côté inférieur (U) pouvant être placé sur un tissu organique, la base présentant une partie de réception (1) et une partie plane (2) qui sont formées d'un seul tenant,
b) un dispositif d'introduction (5) logé de manière mobile depuis la base (1, 2), pouvant être introduit dans le tissu,
c) le dispositif d'introduction (5) pouvant être déplacé par rapport à la base (1, 2) d'une position de protection, dans laquelle une extrémité libre du dispositif d'introduction (5) se trouve derrière le côté inférieur (U) de la base (1, 2), à une position d'introduction, dans laquelle l'extrémité libre du dispositif d'introduction (5) dépasse du côté inférieur (U) de la base (1, 2),
d) et un logement ouvert vers le côté inférieur (U) de la base (1, 2), détachable de la base (1, 2) et recevant au moins l'extrémité libre du dispositif d'introduction (5) dans sa position de protection,
e) une poignée (11, 12, 14) étant reliée de manière amovible à la base (1, 2) et formant le logement amovible (14),
f) la poignée (11, 12, 14) présentant une partie de poignée (11, 12) s'élevant d'un côté supérieur de la base (1, 2) et le logement amovible (14) dépassant latéralement de la partie de poignée (11, 12), le logement amovible (14) entourant la base (1, 2) comme un pot ou une clé, **caractérisée en ce que** la base (1, 2) et un élément articulé (6) forment ensemble une articulation et le dispositif d'introduction (5) dépasse de l'élément articulé (6), de préférence tandis qu'un sens longitudinal du dispositif d'introduction (5) croise ou coupe un axe de déplacement de l'articulation.

2. Tête d'insertion selon la revendication 1, **caractérisée en ce que** la base (1, 2) forme un autre logement (1) ouvert vers le côté inférieur (U) de la base (1, 2) et recevant une section du dispositif d'introduction (5) dans sa position d'introduction et **en ce que** le dispositif d'introduction (5) pénètre par un évidement (4) de l'autre logement (1) dans le logement amovible (14).

3. Tête d'insertion selon la revendication précédente, **caractérisée en ce que** le logement amovible (14) prolonge l'autre logement (1) dans le sens longitudinal du dispositif d'introduction (5), de préférence reçoit l'autre logement (1).

4. Tête d'insertion selon l'une quelconque des deux revendications précédentes, **caractérisée en ce que** l'autre logement (1) présente une surface extérieure qui s'incline de manière précintrée en arrondi vers l'extérieur de son côté supérieur en direction du côté inférieur (U) de la base (1, 2).

5. Tête d'insertion selon l'une quelconque des revendications précédentes, **caractérisée en ce que** le dispositif d'introduction (5) peut être pivoté dans la position d'introduction.

6. Tête d'insertion selon l'une quelconque des revendications précédentes, **caractérisée en ce que** la tête d'insertion comporte une poignée (11, 12, 14) et la poignée (11, 12, 14) comporte un composant de poignée (12) mobile par rapport à la base (1, 2) ou est formée en tant que telle et le composant de poignée mobile (12) est couplé à l'aide d'un engrenage (8, 18) au dispositif d'introduction (5), **en ce qu'**un déplacement ayant lieu par rapport à la base (1, 2) du composant de poignée (12) mobile provoque le déplacement du dispositif d'introduction (5).

7. Tête d'insertion selon la revendication précédente, **caractérisée en ce que** l'engrenage (8, 18) couple le composant de poignée (12) mobile au dispositif d'introduction (5) à l'aide d'un engrenage denté, de préférence d'une roue dentée (8) et d'une crémaillère (18).

8. Tête d'insertion selon la revendication précédente, **caractérisée en ce que** le composant de poignée mobile (12) est relié à une crémaillère (18) qui est formée de préférence sur le composant de poignée mobile (12), et le dispositif d'introduction (5) est relié à une roue dentée (8) se trouvant en engrenage denté avec la crémaillère (18).

9. Tête d'insertion selon l'une quelconque des deux revendications précédentes, **caractérisée en ce que** le dispositif d'introduction (5) est relié de manière à pouvoir pivoter autour d'un axe de rotation et de manière rigide à la torsion à une roue dentée (8) se trouvant en engrenage denté, pouvant tourner autour de l'axe de rotation.

10. Tête d'insertion selon l'une quelconque des revendications 1 à 9, **caractérisée en ce que** la poignée (11, 12, 14) présente un composant de poignée (12) mobile par rapport à la base (1, 2) et une liaison existant entre la base (1, 2) et la poignée (11, 12) se détache lors d'un mouvement du composant de poignée mobile (12).

11. Tête d'insertion selon la revendication précédente, **caractérisée en ce qu'**un premier élément de liaison (9) est formé sur la base (1, 2) et un second élément de liaison (19) est formé sur la poignée (11, 12, 14), **en ce que** les éléments de liaison (9, 19) sont dans un engagement l'un avec l'autre créant la liaison et **en ce qu'**au moins l'un des éléments de liaison (9, 19) peut être déplacé hors de l'engagement de préférence contre une force élastique.

12. Tête d'insertion selon l'une quelconque des deux revendications précédentes, **caractérisée en ce qu'**un premier élément de liaison (9) et un second élément de liaison (19) sont dans un engagement l'un avec l'autre créant la liaison et le composant de poignée mobile (12) touche lors de son mouvement l'un des éléments de liaison (9, 19) et se déplace hors de l'engagement de préférence contre une force élastique.

13. Tête d'insertion selon l'une quelconque des revendications 6 à 12, **caractérisée en ce que** la poignée (11, 12, 14) comporte un autre composant de poignée (11, 14) et le composant de poignée mobile (12) et l'autre composant de poignée (11, 14) peuvent être saisis entre deux doigts d'une main, le composant de poignée mobile (12) pouvant être déplacé contre l'autre composant de poignée (11, 14) en appuyant respectivement l'un des doigts contre l'un des composants (11, 12, 14).

14. Tête d'insertion selon l'une quelconque des revendications 6 à 13, **caractérisée en ce que** le composant de poignée mobile (12) peut être déplacé par rapport à la base (1, 2).

15. Tête d'insertion selon l'une quelconque des revendications 6 à 14, **caractérisée en ce que** le composant de poignée mobile (12) est guidé de manière linéaire.

16. Tête d'insertion selon l'une quelconque des revendications 6 à 15, **caractérisée en ce que** le composant de poignée mobile (12) est mobile au moins sensiblement parallèlement au côté inférieur (U) de la base (1, 2).

17. Tête d'insertion selon l'une quelconque des revendications 6 à 16, **caractérisée en ce que** la poignée (11, 12, 14) comporte un autre composant de poignée (11, 14) qui est immobile par rapport à la base (1, 2) à l'état relié et forme le logement amovible (14).

18. Tête d'insertion selon l'une quelconque des revendications 1 à 17, **caractérisée en ce que** la poignée (11, 12, 14) s'élève d'un côté supérieur de la base (1, 2).

19. Tête d'insertion selon l'une quelconque des revendications précédentes, **caractérisée en ce que** le dispositif d'introduction (5) est flexible et est stabilisé par un dispositif de perforation (15) qui peut être déplacé conjointement avec le dispositif d'introduction (5) de la position de protection à la position d'introduction.

20. Tête d'insertion selon la revendication précédente, **caractérisée en ce que** le dispositif de perforation (15) se relie au logement amovible (14) lors du déplacement du dispositif d'introduction (5) dans la position d'introduction.

21. Tête d'insertion selon l'une quelconque des deux revendications précédentes, **caractérisée en ce que** le dispositif de perforation (15) peut être détaché avec le logement amovible (14) de la base (1, 2) et du dispositif d'introduction (5).

22. Tête d'insertion selon l'une quelconque des revendications précédentes, **caractérisée en ce que** le dispositif d'introduction (5) est allongé dans un sens longitudinal et le sens longitudinal forme avec le côté inférieur (U) de la base (1, 2) un angle aigu inférieur à 50° si l'extrémité libre du dispositif d'introduction (5) avance lors du déplacement dans la position d'introduction au-dessus du côté inférieur (U).

23. Tête d'insertion selon l'une quelconque des revendications précédentes, **caractérisée en ce que** la base (1, 2) loge le dispositif d'introduction (5) de manière à pouvoir pivoter autour d'un axe de rotation et le dispositif d'introduction (5) présente un axe longitudinal qui coupe l'axe de rotation ou le croise à une distance correspondant au maximum à la demi-longueur du dispositif d'introduction (5).
